# EUROPÄISCHE PATENTSCHRIFT

(11) **EP 2 233 483 B1**
(45) Veröffentlichungstag und Bekanntmachung des Hinweises auf die Patenterteilung: **09.05.2012**
(21) Anmeldenummer: 10163200.8
(22) Anmeldetag: 05.08.2003
(51) Int. Cl.: C07D 401/14, C07D 403/12, C07D 239/42

(54) **Indol- oder Benzimidazolderivate zur Modulation der IkB-Kinase und Zwischenprodukte zu deren Herstellung**
Indole or Benzimidazole derivatives as IkB-kinase modulators and intermediates in their preparation.
Dérivés d' indole et d' imidazole en tant que modulateurs de l' IkB-kinase

(30) Priorität: 17.08.2002 DE 10237722
(43) Veröffentlichungstag der Anmeldung: 29.09.2010
(62) Teilanmeldung aus: 03793685.3
(73) Patentinhaber: Sanofi-Aventis Deutschland GmbH, 65926 Frankfurt am Main (DE)
(72) Erfinder: Ritzeler, Olaf, 65926 Frankfurt am Main (DE); Jaehne, Gerhard, 65926 Frankfurt am Main (DE)

(56) Entgegenhaltungen:
- WO-A-01/00610
- WO-A-01/30774
- WO-A1-96/36620
- BONVICINO, GUIDO E. ET AL: "A new type of Smiles rearrangement of N,N-dialkyl-N'-[2-(.omicron.-bromophenoxy) phenyl]-1,3-propanediamines to 2-[N-(3-dialkylaminopropyl)-.omicron.-brom oanilino]phenols" JOC, Bd. 27, Nr. 12, 1962, Seiten 4272-4280, XP002594624

## Beschreibung

Die Erfindung betrifft Indol- oder Benzimidazolderivate, die IκB-Kinase inhibieren, Verfahren zu ihrer Herstellung und die Verwendung derselben als Arzneimittel. Hierin werden Zwischenprodukte für die Herstellung dieser Verbindungen beansprucht, die gleichermaßen einen Teil der Erfindung darstellen. Diejenigen Teile der Beschreibung, die nicht die beanspruchten Zwischenprodukte betreffen, dienen der Erläuterung der Erfindung als Ganzes.

In der Patentanmeldung WO 94/12478 werden unter anderen Indolderivate beschrieben, welche die Blutplättchen-Aggregation inhibieren. In den Patentanmeldungen WO 01 /00610 und WO 01/30774 werden Indol- und Benzimidazolderivate beschrieben, die NFκB modulieren können. NFκB ist ein heterodimerer Transkriptionsfaktor, der eine Vielzahl von Gene aktivieren kann, die unter anderen für proinflammatorische Cytokine wie IL-1, IL-2, TNFα oder IL-6 kodieren. NFκB liegt im Cytosol von Zellen komplexiert mit seinem natürlich vorkommenden Inhibitor IκB vor. Die Stimulation von Zellen, beispielsweise durch Cytokine, führt zur Phosphorylierung und anschließenden proteolytischen Abbau von IκB Dieser proteolytische Abbau führt zur Aktivierung von NFκB, das anschließend in den Kern der Zelle wandert und dort eine Vielzahl von proinflammatorischen Genen aktiviert.
In Erkrankungen wie rheumatoider Arthritis (bei der Entzündung), Osteoarthritis, Asthma, Herzinfarkt, Alzheimer Erkrankungen, Diabetes Typ II, "inflammatory bowel disease" oder Arteriosklerose ist NFκB über das normale Maß hinaus aktiviert. Die Hemmung von NFκB ist auch in der Krebstherapie von Nutzen, da sie dort alleine oder zur Verstärkung der Cytostatika Therapie eingesetzt wird. Es konnte gezeigt werden, dass Arzneimittel wie Glucocorticoide, Salicylate oder Goldsalze, die in der Rheumatherapie eingesetzt werden, an verschiedenen Stellen in die NFκB aktivierende Signalkette inhibierend eingreifen oder direkt mit der Transkription der Gene interferieren.

Der erste Schritt in der genannten Signalkaskade ist der Abbau von IκB. Diese Phosphorylierung wird durch die spezifische IκB-Kinase reguliert. Bisher sind keine Inhibitoren bekannt, die spezifisch IκB-Kinase inhibieren.

Nachteil der bekannten Inhibitoren von IκB-Kinase ist häufig die mangelnde Spezifität der Hemmung für nur eine Klasse von Kinasen. Beispielsweise hemmen die meisten Inhibitoren von IκB-Kinase mehrere verschiedene Kinasen gleichzeitig, weil die katalytischen Domänen dieser Kinasen ähnliche Strukturen aufweisen. Demzufolge wirken die Inhibitoren in unerwünschter Weise auf viele Enzyme, auch solche mit vitaler Funktion ein.

In der Patentanmeldung WO 01/30774 wurden bereits Indolderivate beschrieben, die NFκB modulieren können und eine starke inhibitorische Wirkung auf IκB-Kinase aufweisen. Die in WO 01/30774 offenbarten Verbindungen, die in den Beispielen beschrieben werden, zeigen aber auch eine starke hemmende Wirkung auf andere Kinasen wie cAMP-abhängige Proteinkinase, Proteinkinase C oder Caseinkinase II. Einige dieser Indolderivate zeigen bei einer Verbesserung der Spezifität jedoch die Verringerung der Inhibition von IκB-Kinase. Ferner ist der erzielbare Blutplasmaspiegel mit den in WO 01/30774 offenbarten Verbindungen nicht ausreichend für eine orale Applikation dieser Derivate.

In dem Bestreben wirksame Verbindungen zur Behandlung von rheumatoider Arthritis (bei der Entzündung), Osteoarthritis, Asthma, Herzinfarkt, Alzheimer Erkrankungen, Krebserkrankungen (Potenzierung von Cytotoxica-Therapien) oder Arteriosklerose zu erhalten, wurde nun gefunden, dass die erfindungsgemäßen Indol- und Benzimidazolderivate die obengenannten Nachteile nicht aufweisen. Die erfindungsgemäßen Indol- und Benzimidazolderivate sind starke Inhibitoren der IκB-Kinase , hemmen dabei sehr selektiv Kinasen und weisen einen hohen Blutplasmaspiegel nach oraler Gabe auf.

Die Erfindung betrifft daher die Verbindung der Formel I 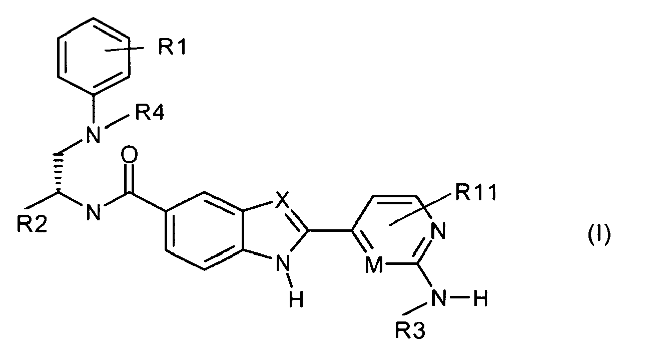 und/oder eine stereoisomere Form der Verbindung der Formel I und/oder ein physiologisch verträgliches Salz der Verbindung der Formel I, wobei
- X und M: gleich oder verschieden sind und unabhängig voneinander für N-Atom oder CH stehen,
- R1 und R11: gleich oder verschieden sind und unabhängig voneinander für
1. Wasserstoffatom,
2. F, Cl, J oder Br,
3. -(C₁-C₄)-Alkyl,
4. -CN,
5. -CF₃,
6. -OR⁵, worin R⁵ für Wasserstoffatom oder -(C₁-C₄)-Alkyl steht,
7. -N(R⁵)-R⁶, worin R⁵ und R⁶ unabhängig voneinander für Wasserstoffatom oder
   -(C₁-C₄)-Alkyl stehen,
8. -C(O)-R⁵, worin R⁵ für Wasserstoffatom oder (C₁-C₄)-Alkyl steht, oder
9. -S(O)ₓ-R⁵, worin x die ganze Zahl Null, 1 oder 2 bedeutet und R⁵ Wasserstoffatom oder -(C₁-C₄)-Alkyl bedeutet, stehen,
- R2 für: 1. einen Heteroarylrest aus der Gruppe 3-Hydroxypyrro-2,4-dion, Imidazol, Imidazolidin, Imidazolin, Indazol, Isothiazol, Isothiazolidin, Isoxazol, 2-Isoxazolidin, Isoxazolidin, Isoxazolon, Morpholin, Oxazol, 1,3,4-Oxadiazol, Oxadiazolidindion, Oxadiazolon, 1,2,3,5-Oxathiadiazol-2-oxid, 5-Oxo-4,5-dihydro-[1,3,4]oxadiazol, 5-Oxo-1,2,4-thiadiazol, Piperazin, Pyrazin, Pyrazol, Pyrazolin, Pyrazolidin, Pyridazin, Pyrimidin, Tetrazol, Thiadiazol, Thiazol, Thiomorpholin, Triazol oder Triazolon, steht, und
   der Heteroarylrest unsubstituiert ist oder ein-, zwei- oder dreifach unabhängig
   voneinander substituiert ist durch
   1.1 -C(O)-R⁵, worin R⁵ für Wasserstoffatom oder -(C₁-C₄)-Alkyl steht,
   1.2 -(C₁-C₄)-Alkyl,
   1.3 -O-R⁵, worin R⁵ für Wasserstoffatom oder -(C₁-C₄)-Alkyl steht,
   1.4 -N(R⁵)-R⁶, worin R⁵ und R⁶ unabhängig voneinander für Wasserstoffatom oder -(C₁-C₄)-Alkyl stehen,
   1.5 Halogen oder
   1.6 Keto-Rest,
2. -C(O)-R⁵, worin R⁵ für Wasserstoffatom oder -(C₁-C₄)-Alkyl steht,
3. -C(O)-OR⁵, worin R⁵ für Wasserstoffatom oder -(C₁-C₄)-Alkyl steht, oder
4. -C(O)-N(R⁷)-R⁸, worin R⁷ und R⁸ unabhängig voneinander für Wasserstoffatom,
   -(C₁-C₄)-Alkyl-OH, -O-(C₁-C₄)-Alkyl oder -(C₁-C₄)-Alkyl stehen,
- R3 für: Wasserstoffatom oder -(C₁-C₄)-Alkyl steht,
R4 für
1. einen Heteroarylrest aus der Gruppe Pyrrol, Furan, Thiophen, Imidazol, Pyrazol, Oxazol, Isoxazol, Thiazol, Isothiazol, Tetrazol, 1,2,3,5-Oxathiadiazol-2-Oxide, Triazolone, Oxadiazolon, Isoxazolon, Oxadiazolidindion, Triazol, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, β-Corbolin und benz-anellierte, cyclopenta-, oder cyclohexa- Derivate dieser Heteroarylreste,
   wobei der Heteroarylrest unsubstituiert ist oder ein-, zwei- oder dreifach unabhängig voneinander substituiert ist durch -(C₁-C₅)-Alkyl, -(C₁-C₅)-Alkoxy, Halogen, Nitro, Amino, Trifluormethyl, Hydroxyl, Hydroxy-(C₁-C₄)-alkyl, Methylendioxy, Ethylendioxy, Formyl, Acetyl, Cyano, Hydroxycarbonyl, Aminocarbonyl oder -(C₁-C₄)-Alkoxycarbonyl, oder
2. einen Arylrest aus der Gruppe Phenyl, Naphthyl, 1-Naphthyl, 2-Naphthyl, Biphenylyl, 2-Biphenylyl, 3-Biphenylyl und 4-Biphenylyl, Anthryl oder Fluorenyl steht, und
   der Arylrest unsubstituiert ist oder ein-, zwei- oder dreifach unabhängig
   voneinander substituiert ist durch -(C₁-C₅)-Alkyl, -(C₁-C₅)-Alkoxy, Halogen, Nitro, Amino, Trifluormethyl, Hydroxyl, Hydroxy-(C₁-C₄)-alkyl, Methylendioxy, Ethylendioxy, Formyl, Acetyl, Cyano, Hydroxycarbonyl, Aminocarbonyl oder
   -(C₁-C₄)-Alkoxycarbonyl.

Die Erfindung betrifft ferner Verbindungen der Formel I, wobei
- X und M: gleich oder verschieden sind und unabhängig voneinander für N-Atom oder CH stehen, R1 und R11 wie oben unter 1. bis 9. definiert sind,
R2 für
1. einen Heteroarylrest aus der Gruppe Imidazol, Isothiazol, Isoxazol, 2-Isoxazolidin, Isoxazolidin, Isoxazolon, 1,3,4-Oxadiazol, Oxadiazolidindion, 1,2,3,5- Oxadiazolon, Oxazol, 5-Oxo-4,5-dihydro-[1,3,4]oxadiazol, Tetrazol, Thiadiazol, Thiazol, Triazol oder Triazolon steht, und der Heteroarylrest unsubstituiert ist oder ein-, zwei- oder dreifach unabhängig voneinander substituiert ist durch
   1.1 Keto-Rest,
   1.2 Halogen oder
   1.3 -(C ₁-C₂)-Alkyl, oder
2. -C(O)-N(R⁷)-R⁸, worin R⁷ und R⁸ unabhängig voneinander für Wasserstoffatom,
   - (C₁-C₄)-Alkyl-OH, -O-(C₁-C₄)-Alkyl oder -(C₁-C₄)-Alkyl stehen,
- R3 für: Wasserstoffatom, Methyl oder Ethyl steht,
- R4 für: 1. einen Heteroarylrest aus der Gruppe der ungesättigten, teilweise gesättigten
   oder vollständig gesättigte Ringe steht, die sich von Pyridin, Pyrazin, Pyrimidin, Pyridazin, Pyrrol, Furan, Thiophen, Imidazol, Pyrazol, Oxazol, Isoxazol, Thiazol, Triazol oder Isothiazol ableiten, wobei der Heteroarylrest unsubstituiert ist oder ein-, zwei- oder dreifach unabhängig voneinander substituiert ist durch -(C₁-C₄)-Alkyl, -(C₁-C₄)-Alkoxy, F, Cl, J, Br, Nitro, Amino, Trifluormethyl, Hydroxyl, Hydroxy-(C₁-C₄)-alkyl, Methylendioxy, Ethylendioxy, Formyl, Acetyl, Cyano, Hydroxycarbonyl, Aminocarbonyl oder -(C₁-C₄)-Alkoxycarbonyl, oder
2. Phenyl steht, und Phenyl unsubstituiert ist oder ein-, zwei- oder dreifach
   unabhängig voneinander substituiert ist durch F, Cl, J, Br, CF₃, -OH,
   -(C₁-C₄)-Alkyl oder -(C₁-C₄)-Alkoxy.

Die Erfindung betrifft ferner die Verbindung
2-(2-Methylamino-pyrimidin-4-yl)-1H-indol-5-carbonsäure [(S)-2-diphenylamino-1-(5-oxo- 4,5-dihydro-[1,3,4]oxadiazol-2-yl)-ethyl]-amid,
2-(2-Methylamino-pyrimidin-4-yl)-1H-indol-5-carbonsäure{1-carbamoyl-2-[(4-fluoro-phenyl)-pyridin-2-yl-amino]-ethyl}-amid,
2-(2-Methylamino-pyrimidin-4-yl)-1H-indol-5-carbonsäure [(S)-1-(5-oxo-4,5-dihydro-1,3,4-oxadiazol-2-yl)-2-(phenyl-pyridin-2-yl-amino)-ethyl]-amid,
2-(2-Amino-pyrimidin-4-yl)-1H-indole-5-carbonsäure {1-carbamoyl-2-[(4-fluorophenyl)-pyridin-2-yl-amino]-ethyl}-amid,
2-(2-Methylamino-pyrimidin-4-yl)-1H-indol-5-carbonsäure [2-[(4-fluoro-phenyl)-pyridin-2-yl-amino]-1-(4H-[1,2,4]triazol-3-yl)-ethyl]-amid,
(S)-2-(2-Methylamino-pyrimidin-4-yl)-1H-indol-5-carbonsäure[1-carbamoyl-2-(phenyl-thiazol-2-yl-amino)-ethyl]-amid,
(S)-2-(2-Methylamino-pyrimidin-4-yl)-1H-indol-5-carbonsäure[1-methoxycarbamoyl-2- (phenyl-pyridin-2-yl-amino)-ethyl]-amid,
2-(2-Amino-pyrimidin-4-yl)-1H-indol-5-carbonsäure{1-carbamoyl-2-[(phenyl)-pyridin-2-yl-amino]-ethyl}-amid,
2-(2-Methylamino-pyrimidin-4-yl)-1H-indol-5-carbonsäure{1-carbamoyl-2-[(phenyl)-pyrimidyl-2-yl-amino]-ethyl}-amid,
2-(2-Methylamino-pyrimidin-4-yl)-1H-indol-5-carbonsäure [1-(2-hydroxy-ethylcarbamoyl)-2-(phenyl-pyrimidin-2-yl-amino)-ethyl]-amid,
(S)-2-{[2-(2-Methylamino-pyrimidin-4-yl)-1H-indol-5-carbonyl]-amino}-3-[phenyl-(4-trifluoromethyl-pyrimidin-2-yl)-amino]-propionsäure,
2-(2-Methylamino-pyrimidin-4-yl)-1H-indol-5-carbonsäure{1-carbamoyl-2-[(4-fluoro-phenyl)-(5-methyl-pyrimidin-2-yl)-amino]-ethyl}-amid,
2-(2-Methylamino-pyrimidin-4-yl)-1H-benzoimidazol-5-carbonsäure-((S)-1-carbamoyl-2- di phenylomino-ethyl)-amid,
2-(2-Methylamino-pyrimidin-4-y)-1H-benzimidazole-5-carbonsäure{1-carbamoyl-2-[(- phenyl)-pyrimidin-2-yl-amino]-ethyl}-amid oder
2-(2-Methylamino-pyrimidin-4-yl)-1H-benzimidazole-5-carbonsäure{1-carbamoyl-2-[(-phenyl)-pyridin-2-yl-amino]-ethyl}-amid.

Unter dem Begriff "Halogen" wird Fluor, Chlor, Brom oder Jod verstanden. Unter dem Begriff
"-(C₁-C₅)-Alkyl" oder "-(C₁-C₅)-Alkoxy" werden Kohlenwasserstoffreste verstanden, deren Kohlenstoffkette geradkettig oder verzweigt ist und 1 bis 5 Kohlenstoffatome enthält wie Methyl, Ethyl, Propyl, n-Butyl, Pentyl oder Tertiär-Butyl. Unter dem Begriff "Heteroarylrest aus der Gruppe der ungesättigten, teilweise gesättigten oder vollständig gesättigte Ringe, die sich von Pyridin, Pyrazin, Pyrimidin, Pyridazin, Pyrrol, Furan, Thiophen, Imidazol, Pyrazol, Oxazol, Isoxazol, Thiazol, Isothiazol ableiten" werden beispielsweise Verbindungen verstanden wie Piperazin, Pyrazolin, Imidazolin, Pyrazolidin, Imidazolidin, Tetrahydropyridin, Isoxazolin, Isoxazolidin, Morpholin, Isothiazolin, Isothiazolidin, Tetrahydro-1,4-thiazin oder Piperidin.

Die Herstellung der Verbindungen der Formel I erfolgt beispielsweise wie es in den Patentanmeldungen WO 01/00610 und WO 01/30774 beschrieben wurde. Die Ausgangsstoffe der chemischen Umsetzungen sind bekannt oder lassen sich nach literaturbekannten Methoden leicht herstellen.

Die Erfindung betrifft ferner ein Verfahren zur Herstellung der Verbindung der Formel I und/oder einer stereoisomeren Form der Verbindung der Formel I und/oder eines physiologisch verträglichen Salzes der Verbindung der Formel I, das dadurch gekennzeichnet ist, dass man
a) eine Verbindung der Formel IV, 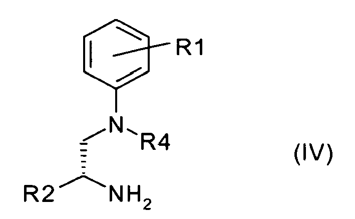 worin R1, R2 und R4 wie in Formel I definiert sind, mit einem Säurechlorid oder einem aktivierten Ester der Verbindung der Formel III, 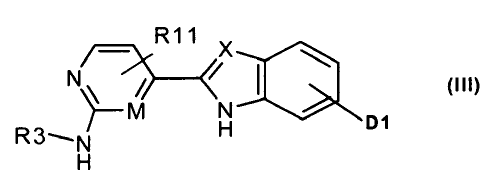 wobei D1 -COOH bedeutet und R11, X, M und R3 wie in Formel I definiert sind, in Gegenwart einer Base oder gegebenenfalls eines wasserentziehenden Mittels in Lösung umsetzt und in eine Verbindung der Formel I überführt,
b) eine nach Verfahren a) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
c) die nach den Verfahren a) oder b) hergestellte Verbindung der Formel I entweder in freier Form isoliert oder im Falle des Vorliegens von sauren oder basischen Gruppen in physiologisch verträgliche Salze umwandelt.

Die Darstellung der Indol- oder Benzimidazolcarbonsäure-Derivate erfolgt nach einer Methode wie sie in Houben-Weyl "Methoden der Org. Chemie", Band E6-2A bzw. E6-2B beschrieben ist.
So lassen sich zur Darstellung der Indol- oder Benzimidazolcarbonsäure-Derivate der Formel III bevorzugt Hydrazinobenzoesäuren und Aryl- oder Heteroarylketone, in Gegenwart von Polyphosphorsäure als Lösungsmittel bei 145°C umsetzen. Die Darstellung der benötigten Hydrazinobenzoesäuren erfolgt nach dem Fachmann geläufigen Methoden z.B. aus den entsprechenden Benzoesäure-anilinen, Aryl- oder Heteroarylketone werden ebenfalls nach dem Fachmann gängigen Methoden z.B. aus den entsprechenden Säurechloriden oder Nitrilen durch Umsetzung mit z.B. Organometall-Verbindungen hergestellt.

Zur Kondensation der Verbindungen der Formel IV mit denen der Formel III verwendet man vorteilhafterweise die dem Fachmann an sich wohlbekannten Kupplungsmethoden der Peptidchemie (siehe z.B. Houben-Weyl, Methoden der Organischen Chemie, Band 15/1 und 15/2, Georg Thieme Verlag, Stuttgart, 1974). Als Kondensationsmittel oder Kupplungsreagenzien kommen Verbindungen wie Carbonyldümidazol, Carbodiimid wie Dicyclohexylcarbodiimid oder Diisopropylcarbodiimid (DIC), das O-((Cyano(ethoxy-carbonyl)-methylen)amino)-N,N,N',N'-tetramethyluronium-tetrafluoroborat (TOTU) oder Propylphosphonsäureanhydrid (PPA) in Frage.

Die Kondensationen können unter Standardbedingungen durchgeführt werden. Bei der Kondensation ist es in der Regel nötig, dass die vorhandenen, nicht reagierenden Aminogruppen durch reversible Schutzgruppen geschützt werden. Gleiches gilt für nicht an der Reaktion beteiligte Carboxylgruppen, die während der Kondensation bevorzugt als -(C₁-C₆)-Alkylester, Benzylester oder tert.-Butylester vorliegen. Ein Aminogruppen-Schutz erübrigt sich, wenn die Aminogruppen noch in Form von Vorstufen wie Nitrogruppen oder Cyanogruppen vorliegen und erst nach der Kondensation durch Hydrierung gebildet werden. Nach der Kondensation werden die vorhandenen Schutzgruppen in geeigneter Weise abgespalten. Beispielsweise können NO₂-Gruppen (Guanidinoschutz in Aminosäuren), Benzyloxycarbonylgruppen und Benzylgruppen in Benzylestern abhydriert werden. Die Schutzgruppen vom tert.-Butyltyp werden sauer abgespalten, während der 9-Fluorenylmethyloxycarbonylrest durch sekundäre Amine entfernt wird.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen zur Prophylaxe und Therapie all solcher Erkrankungen, an deren Verlauf eine verstärkte Aktivität von IκB-Kinase beteiligt ist. Dazu gehören beispielsweise chronische Erkrankungen des Bewegungsapparates wie entzündliche, immunologisch oder stoffwechselbedingte akute und chronische Arthritiden, Arthropathien, rheumatoide Arthritis, oder degenerative Gelenkerkrankungen wie Osteoarthrosen, Spondylosen, Diabetes Typ II, "inflammatory bowel disease", Knorpelschwund nach Gelenktrauma oder längerer Gelenksruhigstellung nach Meniskus- oder Patellaverletzungen oder Bänderrissen oder Erkrankungen des Bindegewebes wie Kollagenosen und Periodontalerkrankungen, Myalgien und Störungen des Knochenstoffwechsels, oder Erkrankungen, die durch eine Überexpression von Tumor Nekrose Faktor alpha (TNFα) oder erhöhte Konzentration an TNFα bedingt sind wie Kachexie, Multiple Sklerose, Schädel-Hirn Trauma, Morbus Crohn und Darmgeschwüre, oder Erkrankungen wie Arteriosklerose, Stenosen, Ulceration, Alzheimers Erkrankungen, Muskelabbau, Krebserkrankungen (Potenzierung von Cytotoxica-Therapien), Herzinfarkt, Gicht, Sepsis, septischer Schock, endotoxischer Schock, virale Infektionen wie Grippe, Hepatitis, HIV-Infektionen, AIDS, oder durch Adenoviren oder Herpesviren verursachte Erkrankungen, parasitische Infektionen wie Malaria oder Lepra, Pilz- oder Hefeinfektionen, Gehirnhautentzündungen, chronische entzündliche Lungenerkrankungen wie chronische Bronchitis oder Asthma, acute respiratory distress syndrome, akute Synovitis, Tuberkulose, Psoriasis, Diabetes, Behandlung von akuten oder chronischen Abstoßungsreaktionen des Organempfängers gegen das verpflanzte Organ, chronische Graft-versus-Host-Erkrankungen und entzündliche Gefäßerkrankungen. Die genannten Erkrankungen können mit den erfindungsgemäß eingesetzten Verbindungen wesentlich spezifischer und mit einem kleineren Nebenwirkungsspektrum behandelt werden, weil im wesentlichen nur IκB-Kinase gehemmt wird.

Die Applikation der erfindungsgemäßen Arzneimittel kann durch orale, inhalative, rektale oder transdermale Applikation oder durch subkutane, intraartikuläre, intraperitoneale oder intravenöse Injektion erfolgen. Bevorzugt ist die orale Applikation.

Die Erfindung betrifft auch ein Verfahren zur Herstellung eines Arzneimittels, das dadurch gekennzeichnet, dass man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylenglykol und Lösungsmittel wie etwa steriles Wasser und ein- oder mehrwertige Alkohole wie Glycerin, genannt. Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt von etwa 50 mg bis 300 mg und bei Injektionslösungen in Ampullenform bis zu etwa 300 mg, vorzugsweise von etwa 10 mg bis 100 mg, betragen. Für die Behandlung eines erwachsenen, etwa 70 kg schweren Patienten sind je nach Wirksamkeit der Verbindung gemäß Formel I, Tagesdosen von etwa 20 mg bis 1000 mg Wirkstoff, bevorzugt von etwa 100 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Endprodukte werden in der Regel durch massenspektroskopische Methoden (FAB-, ESI-MS) bestimmt. Temperaturangaben in Grad Celsius, RT bedeutet Raumtemperatur (22 °C bis 26 °C). Verwendete Abkürzungen sind entweder erläutert oder entsprechen den üblichen Konventionen. Nachfolgend ist die Erfindung an Hand von Beispielen näher erläutert.

### Herstellungsbeispiele

### A) Anilin Darstellung

### A.1.) 2-(p-Fluoranilino)-pyridin (3)

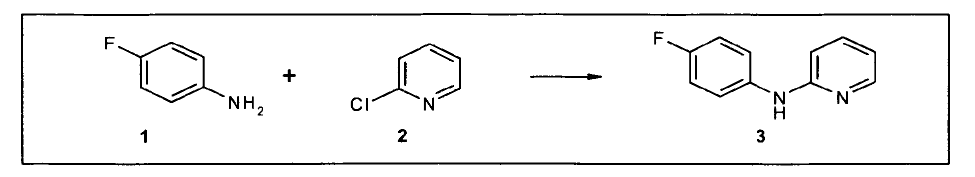

Eine Mischung aus 29.34 g (0.264 mol) 4-Fluoranilin (**1**) und 29.98 g (0.264 mol) 2-Chlorpyridin (2) wurden 2 h auf 150 °C erhitzt. Nach Abkühlen auf RT wurde zwischen 500 ml 1 N NaOH und 500 ml Essigester verteilt. Die wässrige Phase wurde 2 mal mit je 300 ml Essigester extrahiert und die vereinigten organischen Phasen mit Magnesiumsulfat getrocknet. Nach Evaporation des Lösungsmittels wurde der Rückstand in 500 ml Essigester aufgenommen und ca. 40 g Aktivkohle zugegeben. Man ließ 10 Minuten bei RT rühren und filtrierte dann über Kieselgur ab. Die Aktivkohle wurde 4 mal mit je 1 I Essigester nachgewaschen. Das Lösungsmittel wurde im Vakuum (i. V.) entfernt und der Rückstand aus 120 ml Essigester ausgerührt. Der Feststoff wurde abgesaugt und bei 50 °C i. V. getrocknet. Man erhielt 41 g 2-(p-Fluoranilino)-pyridin (**3**); Ausbeute 83%.
Summenformel C₁₁H₉N₂; M.W. = 188.21; MS (M+H) 189.1.
¹H NMR (CDCl₃) 6.68-6.75 (m, 2 H), 6.88 (s, 1 H), 7.05 (t, 2 H), 7.24-7.32 (m, 2 H), 7.43-7.49 (m, 1 H), 8.18 (d, 1 H).

### A.2.) 2-(Anilino)-pyrimidin (6)

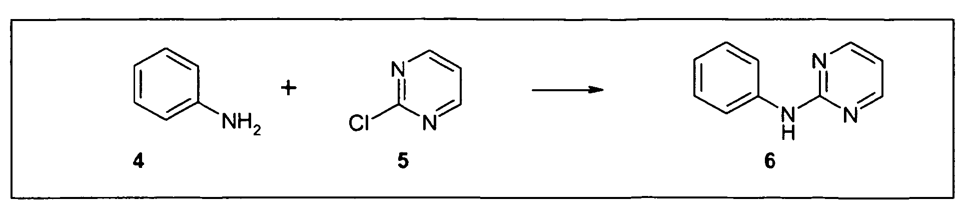

Aus 16.2 g Anilin (**4**) wurden durch analoger Umsetzung wie unter A.1.) beschrieben, mit 2-Chlorpyrimidin (**5**), 9.15 g (31 %) des Anilinopyrimidins **6** erhalten.
Summenformel C₁₀H₉N₃, M.W. = 171.08; MS (M+H) 172.2.

### B.) Aminosäuresynthese über das Z-Serin Lacton 8

### B.1.) (S)-2-Amino-3-diphenylamino-propionsäure-methyl-ester (11)

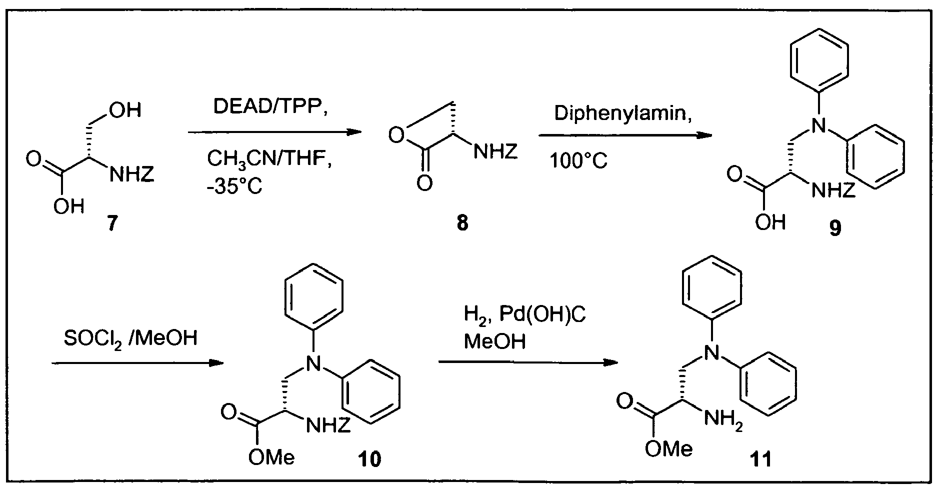

### B.1.1.) N-Benzyloxy-corbonyl-L-serin-β-lacton (8)

54.8 g (0.209 mol) Triphenylphosphin wurden in 600 ml Acetonitril suspendiert und unter Ausschluss von Feuchtigkeit auf -35 °C bis -45 °C gekühlt. Bei dieser Temperatur wurde innerhalb von 50 Minuten tropfenweise 36.4 g (0.209 mol) Azodicarbon-säure-diethylester hinzugegeben. Man rührte 15 Minuten bei -35 °C nach. Zu diesem Gemisch tropfte man dann langsam eine Lösung aus 50 g (0.209 mol) N-Benzyloxycarbonyl-L-serin (7) in 500 ml Acetonitril, so dass die Temperatur nicht über-35°C stieg. Anschließend wurden 12 h bei 5 °C gerührt. Zum Abbruch der Reaktion wurde die Reaktionslösung unter vermindertem Druck vom Lösungsmittel befreit und das Rohprodukt mit
Mitteldruckchromatographie an Kieselgel gereinigt. (DCM/AcCN : 25/1) Nach dem Entfernen des Lösungsmittels erhielt man 20.8 g N-Benzyloxy-carbonyl-L-serin-β-lacton (**8**); Ausbeute 45 %; (siehe auch Org. Synth. 1991 (70) 1ff.) in feinen Nadeln.
Summenformel C₁₁H₁₁NO₄; M.W. = 221.2; MS (M+H) 222.1.
¹H NMR (DMSO-d₆) 4.30 (m, 1H), 4.45 (m, 1H), 5.10 (s, 2H), 5.22 (m, 2H), 7.45 (m, 5H), 8.20 (d, J = 9.8 Hz, 1H).

### B.1.2.) (S)-2-Benzyloxycarbonylamino-3-diphenylamino-propionsäure (9)

5.0 g (22.6 mmol) Serinlacton (5) wurden mit 20 g (118.2mmol) Diphenylamin verrührt und 2h auf 100°C erhitzt. Das Rohprodukt wurde durch Mitteldruckchromatographie an Kieselgel gereinigt. (DCM/Methanol : 9/1, anschließend EE/n-Heptan : 4/1) Nach Entfernen des Lösungsmittels erhielt man 3.65 g (Ausbeute 42%) saubere 2-Benzyloxycarbonylamino-3-diphenylamino-propionsäure (**9**).
Summenformel C₂₃H₂₂N₂O₄; M.W. = 390.44; MS (M+H) 391.2.
¹H NMR (DMSO-d₆) 3.85 (m, 1H), 4.18 (m, 1H), 4.3 (m, 1H), 4.9 (m, 2H), 6.9 (m, 5H), 7.25 (m, 10H).

### B.1.3.) (S)-2-Benzyloxycarbonylamino-3-diphenylamino-propionsäure-methyl-ester (10)

Zu 75 ml Methanol wurden bei -5°C 6.5 ml (89.1 mmol) Thionylchlorid getropft und
15 min gerührt. Anschließend wurde 3.6 g (9.22 mmol) 2-Benzyloxycarbonylamino-3-diphenylamino-propionsäure (**9**) in 75 ml Methanol gelöst zugegeben und weitere 3 Stunden (h) bei Raumtemperatur gerührt. Nach der Evaporierung der Lösungsmittel wurde der Rückstand in Essigester aufgenommen und mit Natriumcarbonat Lösung extrahiert. Die Reinigung mittels Flash-Chromatographie (n-Heptan/Essigester 7:3) lieferte 2.76 g (50% Ausbeute) von 2-Benzyloxycarbonylamino-3-diphenylamino-propionsäure-methyl-ester (**10**).
Summenformel C₂₄H₂₄N₂O₄; M.W. = 404.47; MS (M+H) 405.2.
¹H NMR (DMSO-d₆) 3.58 (s, 3H), 3.95 (m, 1H), 4.18 (m, 1H), 4.4 (m, 1H), 4.95 (m, 2H), 6.9 (m, 6H), 7.3 (m, 9H), 7.85 (d, J = 9.8 Hz, 1H).

### B.1.4.) (S)-2-Amino-3-diphenylamino-propionsäure-methyl-ester (11)

Zur Abspaltung der Z-Schutzgruppe löste man 2.7 g (6.68 mmol) des Z-geschützte Derivates (**10**) in 500 ml Methanol und unter Stickstoff Schutzatmosphäre wurden 100 mg Katalysator (10% Pd(OH)2-C) zugeführt. Anschließend wurde das Inertgas mit einen großen Überschuss Wasserstoff verdrängt und 2 h in der Wasserstoffatmosphäre geschüttelt. Zum Abbruch der Reaktion wurde der Katalysator abfiltriert und das Filtrat eingeengt. Man erhielt 1.65 g (Ausbeute: 91%) 2-Amino-3-diphenylamino-propionsäure-methyl-ester (**11**).
Summenformel C₁₆H₁₈N₂O₂; M.W. = 270.32; MS (M+H) 271.2.
¹H NMR (DMSO-d₆) 3.45 (s, 3H), 3.58 (m, 1H), 3.8 (m, 1H), 3.95 (m, 1H), 6.9 (m, 6H), 7.3 (m, 4H).

### B.2.) Aminosäuresynthese über die Acrylsäure 13

### B.2.1.) Enantiomerentrennung

Die über die Acrylsäure hergestellten racemischen Aminosäuren konnten durch präparative HPLC mit chiralen Phasen wie z. B. Chiralpak AD (Daicel) 100x380, RT, Fluss 300ml/min; in die Enantiomere getrennt werden. Die Enantiomerenreinheit konnte durch analytische HPLC wie Chiralpak-AD-H (Daicel) 4.6x250 , 30°C, flow 1 ml/min, Raumtemperatur) bestimmt werden.

### B.2.2.) (3-(N-4-Fluorphenyl-N-2-pyridyl)-amino)-2-(di-tert.-butyloxycarbonyl)-amino-propionsäure methylester (14)

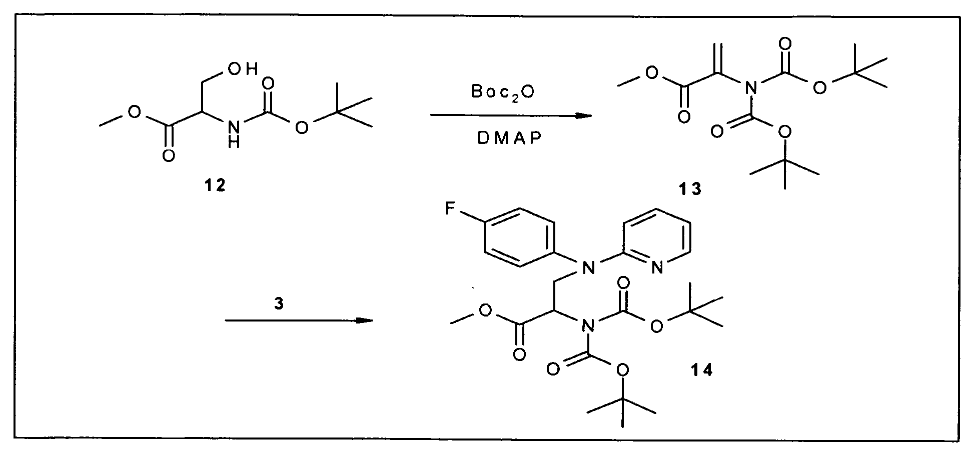

### B.2.2.1.) 2-(di-tert.-Butyloxycarbonyl)-amino-acrylsäure methylester (13)

50 g (0.228 mol) Boc-Serin (**12**) wurden in 300 ml Acetonitril gelöst. Man gab 107 g (0.493 mol) Boc-Anhydrid und 2.64 g (22 mmol) DMAP hinzu. Man ließ über Nacht bei RT rühren, entfernte das Lösungsmittel i. V. und nahm den Rückstand in 500 ml Essigester auf. Die organische Phase wurde mit 500 ml 1 N HCl gewaschen und mit Magnesiumsulfat getrocknet. Durch Kristallisation aus 200 ml Heptan bei - 30 °C wurden nach Absaugen 23 g der Acrylsäure **13** erhalten. Die Mutterlauge wurde eingeengt und der Rückstand in 140 ml Acetonitril gelöst. Man gab 31 g (0.142 mol) Boc-Anhydrid und 1.26 g (10 mmol) DMAP hinzu. Nach Erhitzen auf 50 °C für 8 h wurde das Lösungsmittel i. V. entfernt und der Rückstand in 500 ml Essigester aufgenommen. Die organische Phase wurde mit 400 ml 1 N HCl gewaschen und über Magnesiumsulfat getrocknet. Nach Entfernen des Lösungsmittel i. V. wurden durch Kristallisation aus Heptan weitere 31.5 g des Acrylats **13** erhalten. Ausbeute 54.5 g (0.181 mol) 79 %.
Summenformel C₁₄H₂₃NO₆; M.W. = 301.34; MS ((M*2)+Na⁺) 625.7.
¹H NMR (DMSO-d₆) 1.40 (s, 18 H), 3.74 (s, 3 H), 5.85 (s, 1 H), 6.28 (S, 1 H).B.2.2.2.) (3-(N-4-Fluorphenyl-N-2-pyridyl)-amino)-2-(di-tert.-butyloxycarbonyl)-amino-propionsäure methylester (14)
11.5 g (38.2 mmol) Acrylat **13** wurden mit 7.2 g (38.2 mmol) Anilin **3** und 37.3 g (114 mmol) Cäsiumcarbonat gemischt. Man gab 100 ml Acetonitril hinzu und ließ 2 Tage bei 55°C rühren. Danach ließ man weitere 2 Tage bei RT rühren. Die Feststoffe wurden über Kieselgur abgesaugt und 3 mal mit je 100 ml Acetonitril gewaschen. Die vereinigten organischen Phasen wurden i. V. vom Lösungsmittel entfernt und der Rückstand an Kieselgel mit Heptan/Essigester 4:1 chromatographiert. Ausbeute: 14 g (75 %) 14.
Summenformel C₂₅H₃₂FN₃O₆; M.W. = 489.55; MS (M+H) 490.6.
¹H NMR (CDCl₃) 1.28 (s, 18 H), 3.72 (s, 3 H), 4.25 (dd, 1 H), 4.75 (dd, 1 H), 5.83 (dd, 1 H), 6.22 (d, 1 H), 6.56-6.61 (m, 1 H), 7.05-7.12 (m, 2 H), 7.19-7.26 (m, 3 H), 8.18 (d, 1 H).

### B.2.3.) (3-(N-phenyl-N-2-pyrimidyl)-amino)-2-(di-tert.-butyloxycarbonyl)-amino-propionsäure methylester (15)

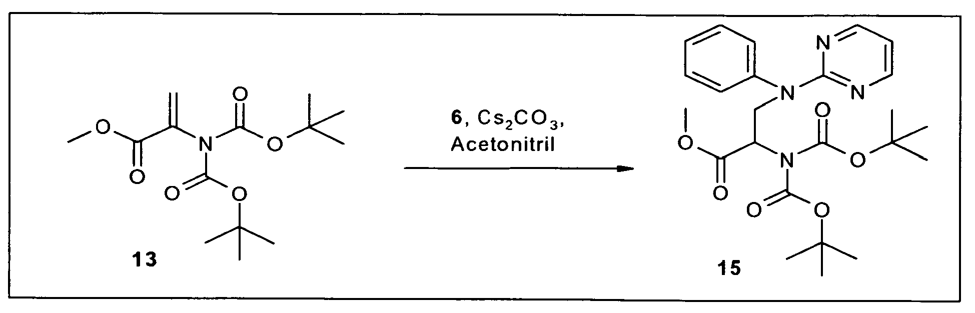

Aus 35 g **6** wurden unter analoger Durchführung, wie unter B.2.2.2.) beschrieben, 3 g (7 %) der Aminosäure **15** erhalten.
Summenformel C₂₄H₃₂N₄O₆; M.W. = 472.23; MS (M+H) 473.1.

### C.) Synthese der heterozyklischen Grundkörper

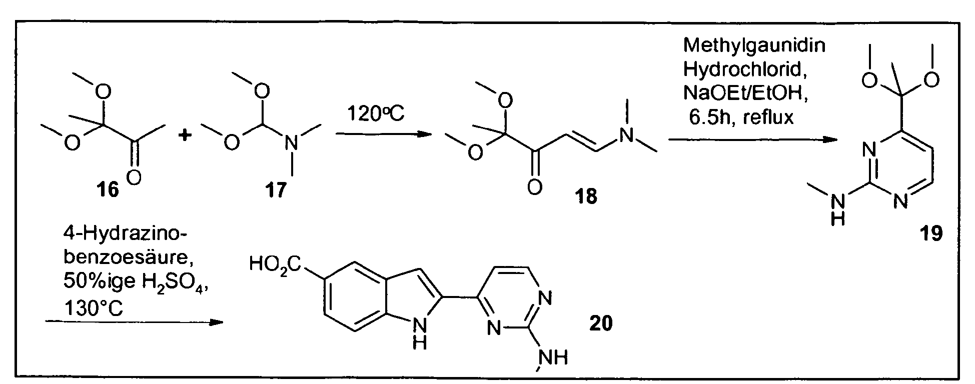

### C.1.) Indol Grundkörper Synthese:

### 2-(2-Methylamino-pyrimidin-4-yl)-1H-indol-5-carbonsäure (20)

### C.1.1.) 1-Dimethylamino-4,4-dimethoxy-pent-1-en-3-on (18)

100 g (0.76 mol) 3,3-Dimethoxy-2-butanon (**16**) wurden mit 90.2 g N,N-Dimethylformamid-dimethylacetal (**17**) (0.76 mol) bei 120°C 48 h gerührt. Das bei der Reaktion entstandene Methanol wurde kontinuierlich von der Reaktionslösung durch Destillation entfernt. Beim Erkalten der Lösung trat spontane Kristallisation ein, welche durch Zugabe von wenig Heptan zur Vollständigkeit gebracht wurde. Man erhielt so 128.24 g Rohprodukt **18** (Ausbeute 90%), welches ohne weitere Reinigung umgesetzt wurde. Summenformel C₉H₁₇NO₃; M.W. = 187.24; MS (M+H) 188.2.
¹H NMR (DMSO-d₆) 1.22 (s, 3H), 2.80 (s, 3H), 3.10 (s, 9H), 5.39 (d, J = 15 Hz, 1H), 7.59 (d, J = 15 Hz, 1H).

### C.1.2.) [4-(1,1-Dimethoxy-ethyl)-pyrimidin-2-yl]-methyl-amin (19)

1.22 g (53 mmol) Natrium wurden in 100 ml absoluten Ethanol gelöst. Dazu wurde unter Rühren 5.8 g (53 mmol) Methylguanidinhydrochlorid und 10 g (53 mmol) 1-Dimethylamino-4,4-dimethoxy-pent-1-en-3-on (**18**) gegeben und 4 h auf Siedehitze erwärmt. Zum Abbruch der Reaktion wurde das Ethanol evaporiert. Das so erhaltene Produkt **19** wurde ohne weitere Reinigung für die Folgereaktion eingesetzt. Ausbeute 11.5 g (58 mmol, quantitativ) Summenformel C₉H₁₅N₃O₂; M.W. = 197.24; MS (M+H) 198.2.
¹H NMR (DMSO-d₆) 1.45 (s, 3H), 2.78 (s, 3H), 3.10 (s, 6H), 6.75 (d, J = 3 Hz, 1H), 7.0 - 7.1 (s(b), 1H), 8.30 (d, J = 3 Hz, 1H).

### C.1.3.) 2-(2-Methylamino-pyrimidin-4-yl)-1H-indol-5-carbonsäure (20)

In 150 ml 50%iger Schwefelsäure wurden bei Raumtemperatur 5 g (25 mmol) [4-(1,1-Dimethoxy-ethyl)-pyrimidin-2-yl]-methyl-amin (**19**) und 3.85 g 4-Hydrazinobenzoesäure unter Rühren gegeben und 4 h auf 130°C erhitzt. Das bei der Reaktion entstandene Methanol wurde kontinuierlich von der Reaktionslösung durch Destillation entfernt. Nach Abkühlen auf 10°C wurde die Reaktionsmischung auf 200 ml Eis gegossen und mit konzentrierter Natronlauge ein pH-Wert von etwa 5.5 eingestellt. Der dabei entstandene Niederschlag von Natriumsulfat und Produktgemisch wurde abfiltriert und der Filter-Rückstand wurde mehrmals mit Methanol extrahiert. Die vereinigten Methanol Extrakte wurden eingeengt und das Produkt **20** durch Flash-Chromatographie (DCM/Methonol 9:1) gereinigt. Ausbeute: 0.76 g (11%)
Summenformel C₁₄H₁₃N₄O₂; M.W. = 268.28; MS (M+H) 269.1.
¹H NMR (DMSO-d₆) 2.95 (s, 3H), 6.90 - 7.10 (s(b), 1H), 7.18 (d, J = 3 Hz, 1H), 7.4 (s, 1H), 7.58 (d, J = 4.5 Hz, 1H), 7.80 (d, J = 4.5 Hz, 1H), 8.30 (s, 1H), 7.80 (d, J = 4.5 Hz, 1H), 8.38 (d, J = 3 Hz, 1H), 11.85 (s, 1H), 12.40 - 12.60 (s(b), 1H).

### C.2.) Benzimidazol Grundkörper Synthese:

### 2-(2-Methylamino-pyrimidin-4-yl)-1H-benzimidazol-5-carbonsäure (25)

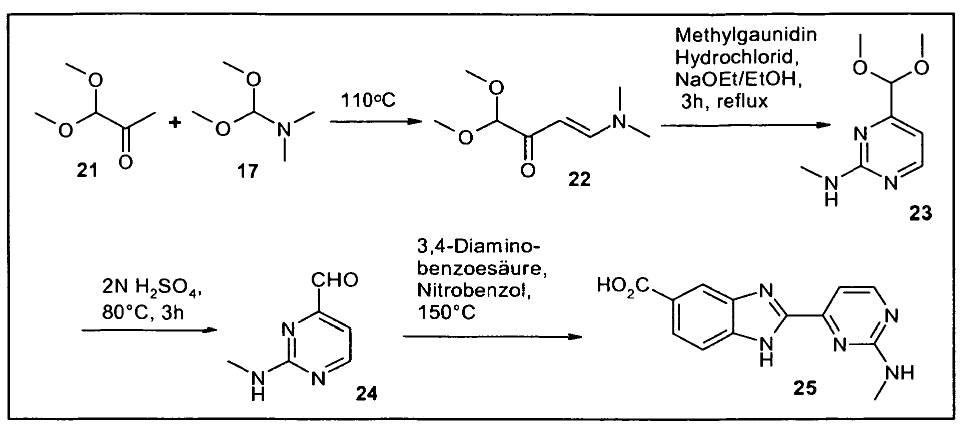

### C.2.1.) 4-Dimethylamin-1,1-dimethoxy-but-3-en-2-on (22)

300 g (307 ml, 2.54 mol) Methylglyoxaldimethylacetal (**21**) wurden mit 303 g (337 ml, 2.54 mol) N,N-Dimethylformamid-dimethylacetal (**17**) bei 110°C 4 Stunden (h) gerührt. Das bei der Reaktion entstandene Methanol wurde kontinuierlich von der Reaktionslösung durch Destillation entfernt. Nach dem Erkalten wurde die Lösung mit Heptan extrahiert und die Lösungsmittel evaporiert. Man erhielt so 303 g Rohprodukt **22** (Ausbeute 70%) welches ohne weitere Reinigung umgesetzt wurde.
Summenformel C₈H₁₅NO₃, M.W. = 173.21; MS (M+H) 174.1
¹H NMR (DMSO-d₆) 2.10 (s, 1H), 2.80 (s, 3H), 3.10 (s, 3H), 3.25 (s, 3H), 3.3 (s, 3H), 4.42 (s, 1H), 5.19 (d (b), J = 12.8 Hz, 1H), 7.60 (d, J = 15 Hz, 1H).

### C.2.2.) (4-Dimethoxymethyl-pyrimidin-2-yl)-methyl-amin (23)

0.33 g (14.4 mmol) Natrium wurden in 50 ml absolutem Ethanol aufgelöst. Dazu wurde unter Rühren 1.57 g (14.4 mmol) Methylguanidinhydrochlorid und 2.48 g (14.4 mmol) 4-Dimethylamin-1,1-dimethoxy-but-3-en-2-on (**22**) gegeben und 3 h auf Siedehitze erwärmt. Zum Abbruch der Reaktion wurde das Ethanol evaporiert. Das so erhaltene Produkt **23** wurde ohne weitere Reinigung eingesetzt. Ausbeute 2.6 g (quantitativ).
Summenformel C₈H₁₃N₃O₂; M.W. = 183.21 ; MS (M+H) 184.1.
¹H NMR (DMSO-d₆) 2.78 (s, 6H), 3.10 (s, 3H), 5.02 (s,1H), 6.62 (d, J = 3 Hz, 1H), 8.30 (d, J = 3Hz, 1H).

### C.2.3.) 2-Methylamino-pyrimidin-4-carbaldehyd (24)

10 g ( 54mmol) (4-Dimethoxymethyl-pyrimidin-2-yl)-methyl-amin (**23**) wurden in 54 ml 2N Schwefelsäure aufgelöst und unter Rühren 3 h auf 80°C erwärmt. Nach dem Erkalten der Reaktion wurde die Reaktionslösung vorsichtig mit festen Na₂CO₃ auf pH von etwa 9 gebracht und mit Ethanol 3 mal extrahiert. Die vereinigten getrockneten Extrakte ergaben nach Evaporierung des Lösungsmittels den Titelaldehyd **24** in 60%iger Ausbeute (4.47g) Summenformel C₆H₇N₃O; M.W. = 137.12 ; MS (M+H) 138.2.
¹H NMR (DMSO-d₆) 2.60 - 2.80 (s(b), 3H), 6.95 (d, J = 3 Hz, 1H), 7.40 - 7.60 (s(b), 1H), 8.55 (d, J = 3 Hz, 1H).

### C.2.4.) 2-(2-Methylamin-pyrimidin-4-yl)-1H-benzoimidazol-5-carbonsäure (25)

4.3 g (31.3 mmol) Methylamin-pyrimidin-4-carbaldehyd (**24**) und 4.8 g (31.1 mmol) 3,4-Diamino-benzoesäure wurden in 300 ml Nitrobenzol 2 h auf 150°C erhitzt. Nach Abkühlen auf 0°C wurde der Niederschlag des Benzimidazols durch Filtration von dem Nitrobenzol getrennt und das Produkt **25** durch Flash-Chromatographie (DCM/Methanol 4 :1) gereinigt. Ausbeute: 2.66 g (32%) Summenformel C₁₃H₂₂N₅O₂; M.W. = 269.28; MS (M+H) 270.2.
¹H NMR (DMSO-d₆) 2.95 (s, 3H), 7.50 (d, J = 3 Hz, 1H), 7.75 (d, J = 4.5 Hz, 1H), 7.90 (d, J = 4.5 Hz, 1H), 8.35 (s, 1H), 8.55 (d, J = 3 Hz, 1H), 8.70 - 9.05 (s(b), 1H).

### D.) Indol Endprodukte

### D.1.) 2-(2-Methylamino-pyrimidin-4-yl)-1H-indol-5-carbonsäure [(S)-2-diphenylamino-1-(5-oxo-4,5-dihydro-[1,3,4]oxadiazol-2-yl)-ethyl]-amid (28)

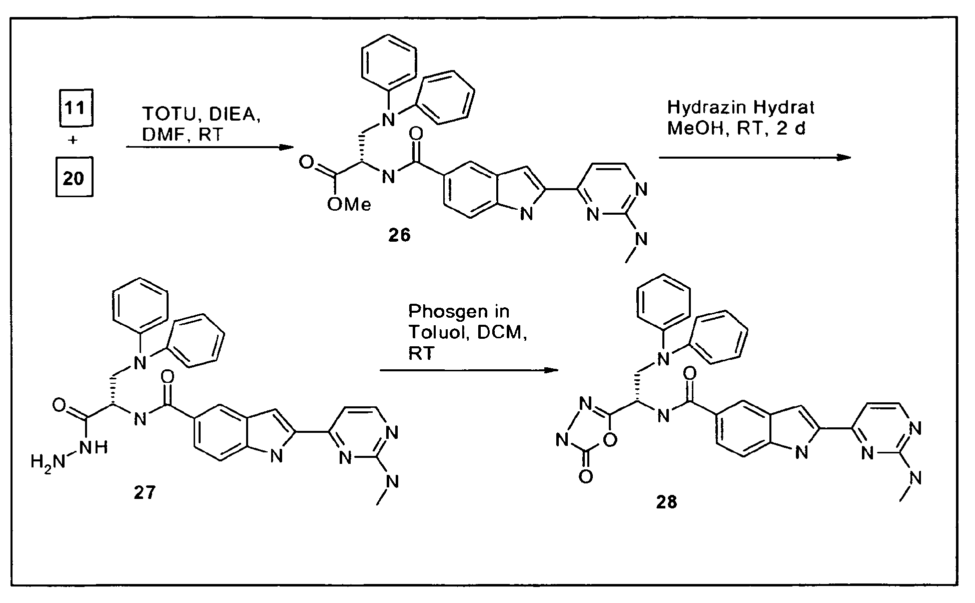

### D.1.1.) 3-Diphenylamino-2-{[2-(2-methylamino-pyrimidin-4-yl)-1H-indol-5-carbonyl]-(S)-amino}- propionsäure methyl ester (26)

5.0 g (18.64 mmol) 2-(2-Methylamino-pyrimidin-4-yl)-1H-indol-5-carbonsäure (**20**) wurden in 1.2 1 DMF gelöst und nacheinander mit 7.9 g (24.08 mmol) TOTU und 7.9 ml (46.45 mmol) Ethyldiisopropylamin versetzt. Man rührte 20 min. bei 5°C und gab zu der Lösung 0.73 g (3.28 mmol) (S)-2-Amino-3-diphenylamino-propionsäure-methyl-ester (**11**) hinzu. Nach 15 h Rühren engte man unter vermindertem Druck ein, nahm den Rückstand in n-Butanol auf und extrahierte die organische Phase zur Abtrennung von Nebenprodukten mit gesättigter Natriumhydrogencarbonatlösung. Nach dem Trocknen mit MgSO₄ und Einengen der organischen Phase wurde der Methylester der Titelverbindung **26** durch Flash-Chromatographie an Kieselgel (DCM:MeOH= 19:1) isoliert.
Ausbeute: 4.3 g (98%)
Summenformel C₃₀H₂₈N₆O₃; M.W. = 520.22; MS (M+H) 521.3.
¹H NMR (DMSO-d₆) 2.95 (s(b), 3H), 3.60 (s, 3H), 4.19 - 4.58 (m, 2H), 4.85 (q,1H), 6.90 - 7.10 (m, 7H), 7.18 (d, J = 3 Hz, 1H), 7.25 - 7.40 (m, 5H), 7.50 (d, J = 4.5 Hz, 1H), 7.65 (d, J = 4.5 Hz, 1H), 8.05 (s, 1H), 8.35 (d, J = 3 Hz, 1H), 8.70 (d, J = 3.75 Hz, 1H), 11.85 (s, 1H).

### D.1.2.) 2-(2-Methylamino-pyrimidin-4-yl)-1H-indol-5-carbonsäure ((S)-2-diphenylamino-1- hydrazin-carbonyl-ethyl)-amid (27)

1.0 g (1.92 mmol) 3-Diphenylamino-2-{[2-(2-methylamino-pyrimidin-4-yl)-1H-indol-5-carbonyl]-(S)-amino}- propionsäure methyl ester (**26**) wurden in 10 ml Methanol gelöst, mit 0.48 g (9.95 mmol) Hydrazinhydrat versetzt und 15 h bei RT gerührt. Der Niederschlag des Produktes (0.3 g) wurde durch Filtration von der Mutterlauge getrennt. Aus der eingeengten Mutterlauge wurde durch Flash-Chromatographie an Kieselgel (DCM:MeOH= 19:1) weiteres Hydrazid **27** (0.1 g) isoliert. Ausbeute: 0.4 g (40%)
Summenformel C₂₉H₂₈N₈O₂; M.W. = 520.6; MS (M+H) 521.4.
¹H NMR (DMSO-d₆) 2.95 (s(b), 3H), 4.02 - 4.58 (m, 2H), 4.4 (s, 2H), 4.85 (q,1H), 6.90 - 7.10 (m, 7H), 7.18 (d, J = 3 Hz, 1H), 7.20 - 7.45 (m, 5H), 7.50 (d, J = 4.5 Hz, 1H), 7.62 (d, J = 4.5 Hz, 1H), 7.99 (s, 1H), 8.25 (d, J = 3 Hz, 1H), 8.35 (s(b), 1H), 9.30 (s, 1H), 11.70 (s, 1H).

### D.1.3.) 2-(2-Methylamino-pyrimidin-4-yl)-1H-indol-5-carbonsäure [(S)-2-diphenylamino-1-(5-oxo- 4,5-dihydro-[1,3,4]oxadiazol-2-yl)-ethyl]-amid (28)

200 mg (0.384 mmol) 2-(2-Methylamino-pyrimidin-4-yl)-1H-indol-5-carbonsäure ((S)-2-diphenylamino-1-hydrazincarbonyl-ethyl)-amid (**27**) wurden in 20 ml Methylenchlorid suspendiert und bei 0°C wurde eine 20%ige Phosgen Lösung in Toluol (0.398 mmol) unter Rühren zugetropft. Es wurde weitere 15 h bei Raumtemperatur gerührt und das Lösungsmittel eingeengt. Das Oxadiazolon **28** wurde anschließend durch Flash-Chromatographie an Kieselgel (DCM:MeOH= 9:1) isoliert. Ausbeute: 160 mg (76%)
Summenformel C₃₀H₂₆N₈O₃; M.W. = 546.6; MS (M+H) 547.3.
¹H NMR (DMSO-d₆) 2.95 (s(b), 3H), 4.02 - 4.58 (m, 2H), 4.85 (q,1H), 6.90 - 7.10 (m, 7H), 7.15 (d, J = 3 Hz, 1H), 7.20 - 7.40 (m, 6H), 7.52 (d, J = 4.5 Hz, 1H), 7.68 (d, J = 4.5 Hz, 1H), 8.10 (s,1H), 8.92 (d, J = 3 Hz, 1H), 11.78 (s, 1H), 12.15 - 12.40 (s(b), 1H).

### D.2.) 2-(2-Methylamino-pyrimidin-4-yl)-1H-indole-5-carboxylic acid {1 - carbamoyl-2-[(4-fluoro- phenyl)-pyridin-2-yl-amino]-ethyl}-amide (30)

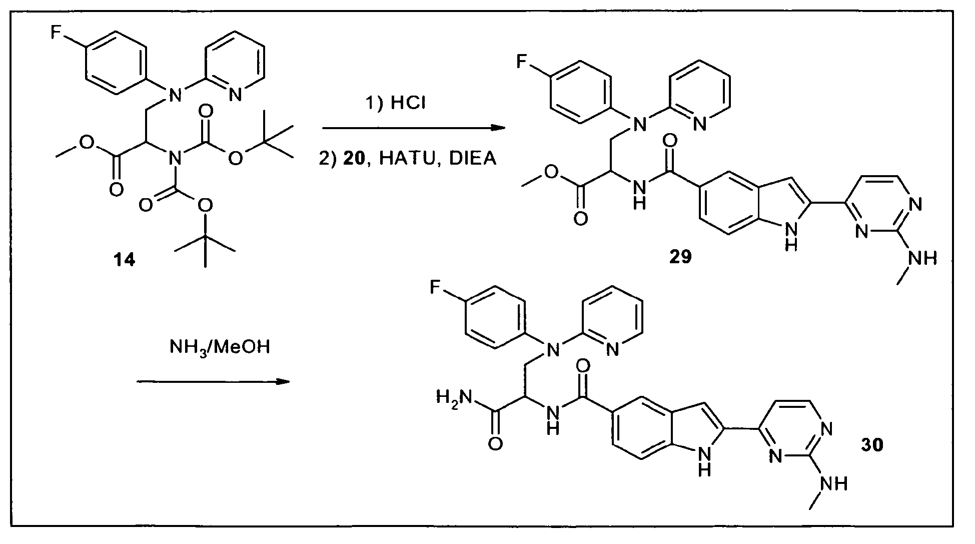

### D.2.1.) 3-[(4-Fluoro-phenyl)-pyridin-2-yl-amino]-2-{[2-(2-methylamino-pyrimidin-4-yl)-1H-indole-5-carbonyl]-amino}-propionic acid methyl ester (29)

0.75 g (1.53 mmol) **14** wurden in 10 ml Dioxan gelöst und auf 0 °C gekühlt. Man gab 10 ml 4 N HCl in Dioxan hinzu, ließ innerhalb von 2 h auf RT kommen und 12 h nachrühren. Das Lösungsmittel wurde i. V. entfernt. Der Rückstand wurde in 10 ml DMF aufgenommen (**Lösung A**). 617 mg der Säure **20** wurden in 20 ml DMF gelöst und auf 0 °C gekühlt. Man gab 1.05 g HATU sowie 1.6 ml DIEA hinzu.

Nach Rühren für 40 Minuten bei 0 °C wurde **Lösung A** hinzugegeben. Man ließ auf RT kommen und rührte 4 h nach. Das Lösungsmittel wurde i. V. entfernt und der Rückstand zwischen 100 ml gesättigter (ges.) NaHCO₃-Lösung und 100 ml Essigester verteilt. Die wässrige Phase wurde 3 mal mit je 50 ml Essigester extrahiert und die vereinigten organischen Phasen mit 100 ml gesättigte NaCl-Lösung gewaschen. Die organische Phase wurde mit Magnesiumsulfat getrocknet. Die Lösungsmittel wurden i. V. entfernt und der Rückstand an Kieselgel mit Heptan/Essigester 1:3 chromatographiert. Man erhielt 560 mg (68 %) des Esters **29.** Summenformel C₂₉H₂₆FN₇O₃; M.W. = 539.57; MS (M+H) 540.2.

### D.2.2.) 2-(2-Methylamino-pyrimidin-4-yl)-1H-indole-5-carboxylic acid {1-carbamoyl-2-[(4-fluoro- phenyl)-pyridin-2-yl-amino]-ethyl}-amide (30)

320 mg (0.593 mmol) des Esters **29** wurden bei 0 °C in 50 ml Ammoniak gesättigtem Methanol gelöst. Man ließ 24 h rühren und auf RT kommen. Die Lösungsmittel wurden i. V. entfernt und der Rückstand aus 5 ml Essigester ausgerührt. Der Feststoff wurde abgesaugt und bei 50 °C i. V. getrocknet. Man erhielt 270 mg (87 %) des Amids **30**.
Summenformel C₂₈H₂₅FN₈O₂; M.W. = 524.56; MS (M+H) 525.2.
¹H NMR (DMSO-d₆) 2.45 (s, 3 H), 4.10 (d, 1 H), 4.52-4.66 (m, 2 H), 6.26 (d, 1 H), 6.77 (t, 1 H), 7.02 (bs, 1 H), 7.09-7.17 (m, 2 H), 7.22-7.32 (m, 5 H), 7.38-7.46 (m, 1 H), 7.47-7.58 (m, 3 H), 7.92 (s, 1 H), 8.27-8.36 (M, 2 H), 8.59 (d, 1 H), 11.70 (s, 1 H).

### D.3.) 2-(2-Methylamino-pyrimidin-4-yl)-1H-indole-5-carboxylic acid [(S)-1-(5-oxo-4,5-dihydro-1,3,4-oxadiazol-2-yl)-2-(phenyl-pyridin-2-yl-amino)-ethyl]-amide (33)

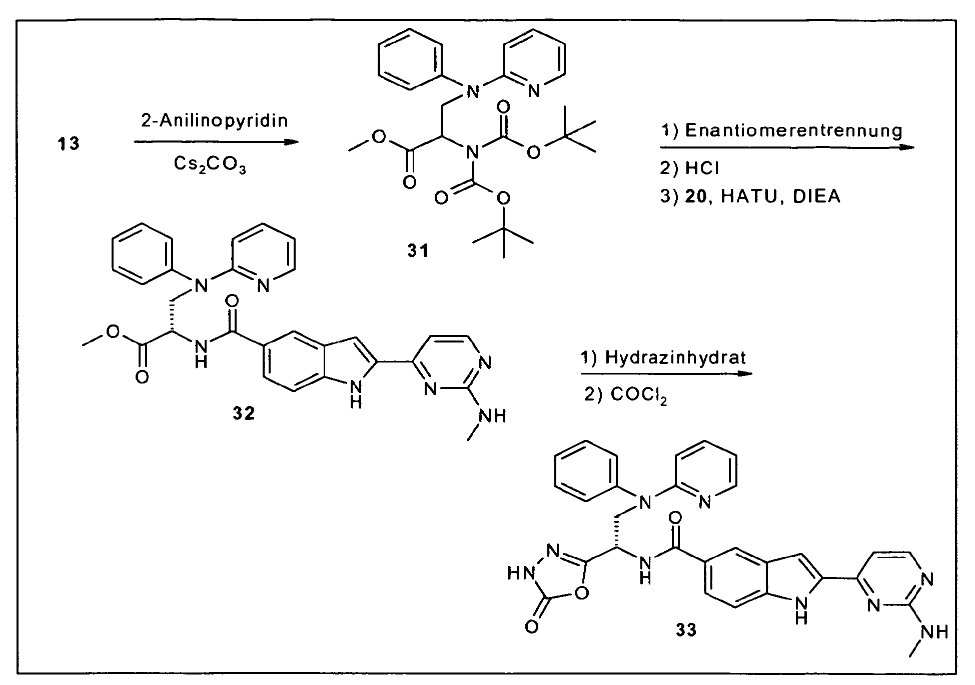

### D.3.1.) (3-(N-Phenyl-N-2-pyridyl)-amino)-2-(di-tert.-butyloxycarbonyl)-amino-propionsäure methylester (31)

4.96 g (16.5 mmol) Acrylat **13** wurden mit 5.6 g (32.9 mmol) 2-Anilinopyridin und 32.16 g (98.7 mmol) Cäsiumcarbonat gemischt. Man gab 50 ml Acetonitril hinzu und ließ 2 Tage bei 45 °C rühren. Der Feststoff wurde über Kieselgur abgesaugt und 3 mal mit je 100 ml Acetonitril gewaschen. Die vereinigten organischen Phasen wurden eingeengt und der Rückstand an Kieselgel mit Heptan/Diethylether 1:1 chromatographiert. Man erhielt 5.66 g (73 %) des Esters **31**. Summenformel C₂₅H₃₃N₃O₆; M.W. = 471.56; MS (M+H) 472.2.

### D.3.2.) Die Enantiomerentrennung erfolgte wie unter B.2.1.) beschrieben.

### D.3.3.) 2-{[2-(2-Methylamino-pyrimidin-4-yl)-1H-indole-5-carbonyl]-amino}-3-(phenyl-pyridin-2-yl-amino)-propionic acid methyl ester (32)

2.9 g des S-Enantiomeren von **31** wurden in 30 ml Dioxan gelöst und auf 0°C gekühlt. Man gab 30 ml 4 N HCl in Dioxan hinzu, ließ auf RT kommen und 12 h nachrühren. Das Lösungsmittel wurde i. V. entfernt. Der Rückstand wurde in 30 ml DMF aufgenommen (**Lösung A**).
2.47 g (9.2 mmol) der Säure **20** wurden in 50 ml DMF gelöst und auf 0 °C gekühlt. Man gab 4.21 g HATU sowie 6.4 ml DIEA hinzu. Nach Rühren für 45 Minuten bei 0°C ließ man auf RT kommen und gab **Lösung A** hinzu. Man ließ 12 h bei RT rühren. Das Lösungsmittel wurde i. V. entfernt und der Rückstand zwischen 300 ml ges. NaHCO₃-Lösung und 300 ml Essigester verteilt. Die wässrige Phase wurde 3 mal mit je 100 ml Essigester extrahiert und die vereinigten organischen Phasen mit 400 ml ges. NaCl-Lösung gewaschen. Die organische Phase wurde mit Magnesiumsulfat getrocknet. Die Lösungsmittel wurden i. V. entfernt und der Rückstand an Kieselgel mit Heptan/Essigester 1:3 chromatographiert. Man erhielt 1.78 g (55 %) des Esters **32**.
Summenformel C₂₉H₂₇N₇O₃; M.W. = 521.58; MS (M+H) 522.2.

### D.3.4.) 2-(2-Methylamino-pyrimidin-4-yl)-1H-indole-5-carboxylic acid [(S)-1-(5-oxo-4,5-dihydro-1,3,4-oxadiazol-2-yl)-2-(phenyl-pyridin-2-yl-amino)-ethyl]-amide (33)

1.78 g (3.4 mmol) Ester **32** wurden in 30 ml MeOH gelöst. Man gab 0.83 ml Hydrazinhydrat hinzu und ließ 5 h bei 40 °C rühren. Danach wurden weitere 1.6 ml Hydrazinhydrat zugegeben und 15 h bei RT gerührt. Die Lösungsmittel wurden i. V. entfernt und der Rückstand in 80 ml Dichlormethan aufgenommen. Man gab 3.2 ml einer 20 %-igen Lösung von Phosgen in Toluol hinzu und ließ 3 Tage rühren. Danach wurde das Lösungsmittel i. V. entfernt und der Rückstand zwischen 80 ml Wasser und 80 ml Essigester verteilt. Dabei fiel ein Feststoff aus der abgesaugt wurde. Die organische Phase wurde eingeengt und der Rückstand mit dem Feststoff vereinigt und an Kieselgel mit Heptan/Essigester 1:5 chromatographiert. Man erhielt 390 mg (21 %) des Oxadiazolons **33**. Summenformel C₂₉H₂₅N₉O₃; M.W. = 547.58; MS (M+H) 548.2
¹H NMR (DMSO-d₆) 2.96 (s, 3 H), 4.30 (dd, 1 H), 4.67 (dd, 1 H), 5.40 (dd, 1 H), 6.32 (d, 1 H), 6.70-6.75 (m, 1 H), 6.98 (bs, 1 H), 7.16 (d, 1 H), 7.22-7.33 (m, 4 H), 7.38-7.46 (m, 3 H), 7.52 (d, 1 H), 7.63 (d, 1 H), 8.08 (s, 1 H), 8.21 (d, 1 H), 8.31-8.35 (m, 1 H), 9.00 (d, 1 H), 11.72 (s, 1 H), 12.15 (s, 1 H).

### D.4.) 2-(2-Amino-pyrimidin-4-yl)-1H-indole-5-carboxylic acid {1-carbamoyl-2-[(4-fluoro- phenyl)-pyridin-2-yl-amino]-ethyl}-amide (35)

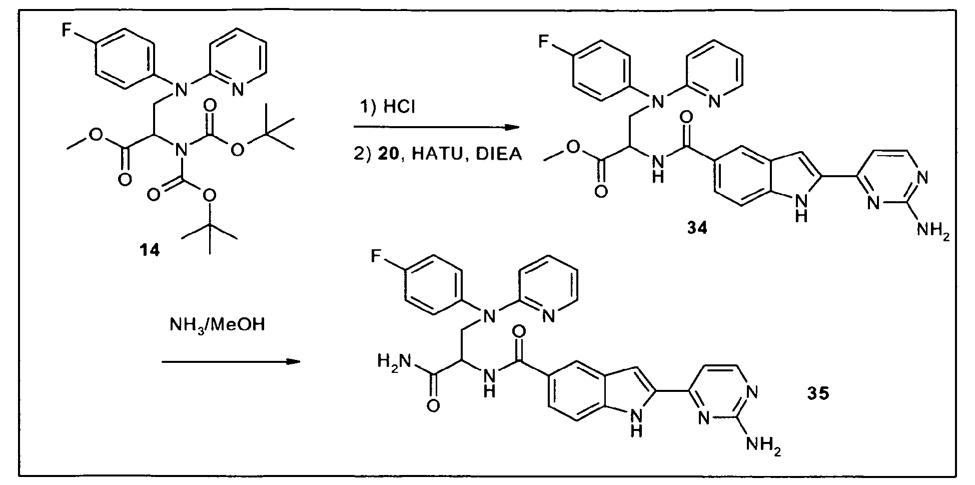

### D.4.1.) 3-[(4-Fluoro-phenyl)-pyridin-2-yl-amino]-2-{[2-(2-amino-pyrimidin-4-yl)-1H-indole- 5-carbonyl]-amino}-propionic acid methyl ester (34)

Aus 750 mg **14** wurden unter analoger Durchführung wie unter D.2.1.) beschrieben, 370 mg (46 %) des Methylesters **34** erhalten.
Summenformel C₂₈H₂₄FN₇O₃; M.W. = 525.55; MS (M+H) 526.2.

### D.4.2.) 2-(2-Amino-pyrimidin-4-yl)-1H-indole-5-carboxylic acid {1-carbamoyl-2-[(4-fluoro- phenyl)-pyridin-2-yl-amino]-ethyl}-amide (35)

Aus 150 mg **34** wurden unter analoger Durchführung wie unter D.2.2.) beschrieben, 95 mg (65 %) des Amids **35** erhalten.
Summenformel C₂₇H₂₃FN₈O₂; M.W. = 510.54; MS (M+H) 511.2.
¹H NMR (DMSO-d₆) 4.08-4.17 (m, 1 H), 4.54-4.65 (m, 2 H), 6.29 (d, 1 H), 6.54 (s, 2 H), 6.74-6.80 (m, 1 H), 7.10 (s, 1 H), 7.18 d, 1 H), 7.22-7.31 (m, 4 H), 7.38-7.56 (m, 6 H), 7.92 (s, 1 H), 8.29-8.35 (m, 2 H), 8.74 (d, 1 H), 11.80 (s, 1 H).

### D.5.) 2-(2-Methylamino-pyrimidin-4-yl)-1H-indole-5-carboxylic acid [2-[(4-fluorophenyl)-pyridin-2-yl-amino]-1-(4H-[1,2,4]triazol-3-yl)-ethyl]-amide (36)

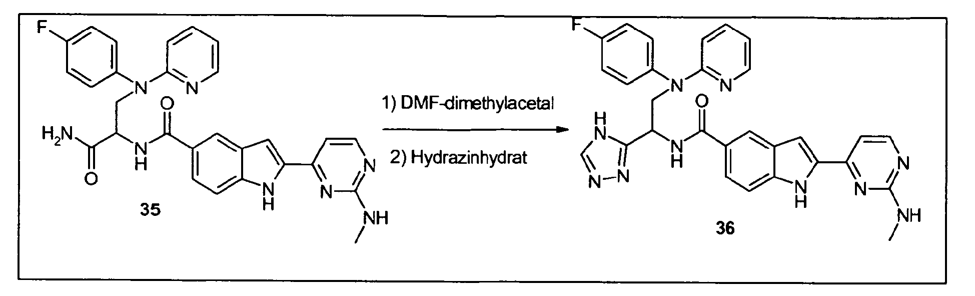

30 mg (0.25 mmol) Amid **35** wurden in 10 ml DMF gelöst. Man gab 40 µl DMFdimethylacetal hinzu und erhitzte 4 h auf 90°C. Das Lösungsmittel wurde i. V. entfernt und der Rückstand in 3.5 ml Essigsäure aufgenommen. Nach Zugabe von 27 µl Hydrazinhydrat ließ man 18 h rühren. Das Lösungsmittel wurde i. V. entfernt und der Rückstand mittels präparativer HPLC gereinigt. Man erhielt 84 mg (50 %) des Triazols **36**.
Summenformel C₂₉H₂₅FN₁₀O; M.W. = 548.59; MS (M+H) 549.2.
¹H NMR (DMSO-d₆) 3.04 (s, 3 H), 4.36-4.43 (m, 1 H), 4.49-4.59 (m, 1 H), 5.60-5.67 (m, 1 H), 6.50 (d, 1 H), 6.78 (t, 1 H), 7.17-7.37 (m, 7 H), 7.45-7.65 (m, 4 H), 8.02 (s, 1 H), 8.19 (d, 1 H), 8.35 (d, 1 H), 8.39 (d, 1 H), 11.85 (s, 1 H).

### D.6.) S-2-(2-Methylamino-pyrimidin-4-yl)-1H-indole-5-carboxylic acid [1-carbamoyl-2-(phenyl- thiazol-2-yl-amino)-ethyl]-amide (42)

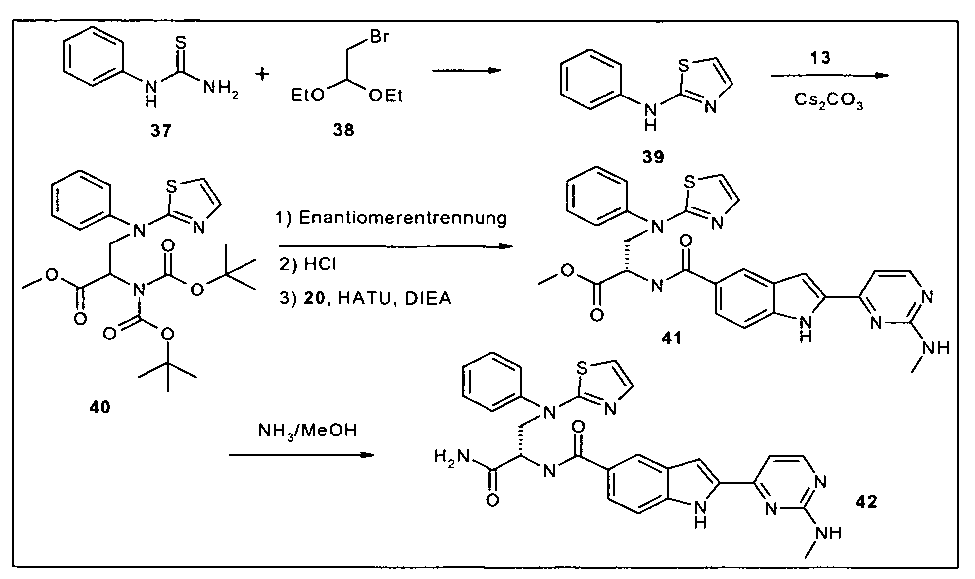

### D.6.1.) Phenyl-thiazol-2-yl-amine (39)

10 g (65.7 mmol) Phenylthioharnstoff **37** wurden in 100 ml Essigsäure gelöst. Man gab 9.9 ml des Acetals **38** hinzu und erhitzte für 2 h auf 100 °C. Das Lösungsmittel wurde i. V. entfernt und der Rückstand zwischen 300 ml 1 N NaOH und 300 ml Essigester verteilt. Die wässrige Phase wurde 2 ml mit je 100 ml Essigester extrahiert und die vereinigten organischen Phasen mit Magnesiumsulfat getrocknet. Das Lösungsmittel wurde entfernt und der Rückstand aus 50 ml Diisopropylether ausgerührt. Der Feststoff wurde abgesaugt und bei 50°C i. V. getrocknet. Man erhielt 2.5 g Anilin **39.** Die Diisopropylether Mutterlauge wurde eingeengt und der Rückstand an Kieselgel mit Heptan/Essigester 2:1 chromatographiert. Dadurch wurden weitere 3.5 g **39** erhalten. Ausbeute: 6.0 g (52 %).
Summenformel C₉H₈N₂S; M.W. = 176.24; MS (M+H) 177.1.

### D.6.2.) (3-(N-Phenyl-N-2-thiazolyl)-amino)-2-(di-tert.-butyloxycarbonyl)-amino-propionsäure methylester (40)

Aus 3.8 g (12.5 mmol) Acrylat **13,** 2.2 g (12.5 mmol) Anilin **39** und 20 g Cäsiumcarbonat wurden analog der Durchführung wie unter D.3.1.) beschrieben, 4.5 g (75 %) Ester **40** erhalten.
Summenformel C₂₃H₃₁N₃O₆S; M.W. = 477.58; MS (M+H) 478.2.

### D.6.3.) Die Enantiomerentrennung erfolgte wie unter B.2.1.) beschrieben.

### D.6.4.) S-2-{[2-(2-Methylamino-pyrimidin-4-yl)-1H-indole-5-carbonyl]-amino}-3-(phenyl-thiazol-2-yl-amino)-propionic acid methyl ester (41)

Aus 1.07 g (2.2 mmol) Ester **40** und 901 mg (3.3 mmol) Säure **20** wurden analog der Durchführung wie unter D.3.3.) beschrieben, 640 mg (55 %) **41** erhalten. Summenformel C₂₇H₂₅N₇O₃S; M.W. = 527.61; MS (M+H) 528.1.

### D.6.4.) S-2-(2-Methylamino-pyrimidin-4-yl)-1H-indole-5-carboxylic acid [1-carbamoyl-2-(phenyl-thiazol-2-yl-amino)-ethyl]-amide (42)

Aus 500 mg (0.95 mmol) **41** wurden analog der Durchführung wie unter D.2.2.) beschrieben, 340 mg (70 %) des Amids **42** erhalten.
Summenformel C₂₆H₂₄N₈O₂S; M.W. = 512.60; MS (M+H) 513.3.
¹H NMR (DMSO-d₆) 2.97 (s, 3 H), 4.23-4.30 (M, 1 H), 4.39-4.48 (M, 1 H), 4.71-4.78 (m, 1 H), 6.78 (d, 1 H), 7.16 (d, 1 H), 7.28-7.35 (m, 3 H), 7.37-7.60 (m, 7 H), 7.98 (s, 1 H), 8.33 (d, 1 H), 8.62 (d, 1 H), 11.70 (s, 1 H).

### D.7.)S-2-(2-Methylamino-pyrimidin-4-yl)-1H-indole-5-carboxylic acid [1-methoxycarbamoyl-2-(phenyl-pyridin-2-yl-amino)-ethyl]-amide (43)

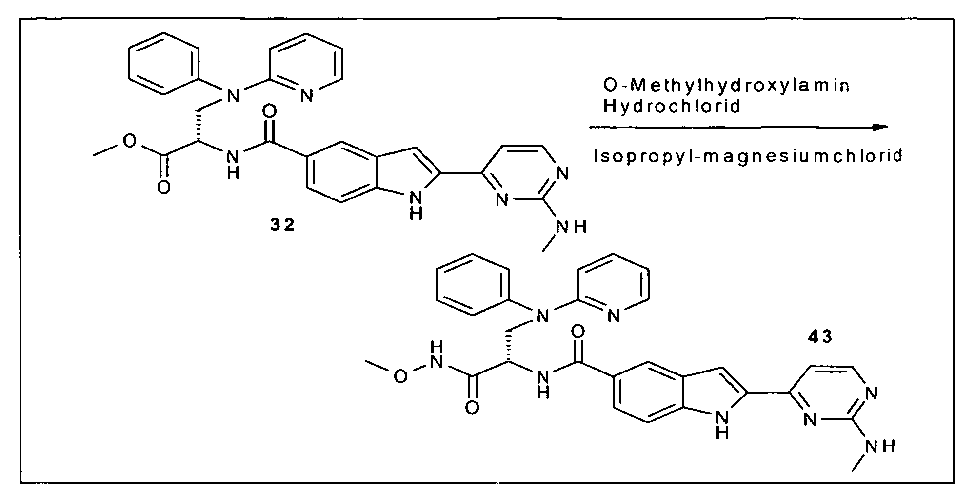

80 mg (0.95 mmol) O-Methylhydroxylamin Hydrochlorid wurden in 10 ml THF gelöst und auf
- 40°C gekühlt. Man tropfte 0.95 ml (1.9 mmol) einer 2 M Lösung von Isoprppylmagnesium-chlorid in THF zu. Innerhalb von 1 h ließ man auf - 20°C kommen. Dann wurde eine Lösung von 100 mg (0.19 mmol) Ester **32** in 3 ml THF zugetropft. Innerhalb von 4 h ließ man auf RT kommen und beendete die Reaktion durch Zugabe von 5 ml Wasser. Das THF wurde i. V. entfernt und der Rückstand zwischen 20 ml gesättigte Ammoniumchlorid Lösung und 20 ml Essigester verteilt. Die wässrige Phase wurde 3 mal mit je 20 ml Essigester extrahiert und die vereinigten organischen Phasen mit Magnesiumsulfat getrocknet. Das Lösungsmittel wurde i. V. entfernt und der Rückstand durch präparative HPLC gereinigt. Man erhielt 60 mg (61 %) des Methylhydroxamats **43**.
Summenformel C₂₉H₂₈N₈O₃; M.W. = 536.60; MS (M+H) 537.2.
¹H NMR (DMSO-d₆) 2.95 (s, 3 H), 3.52 (s, 3 H), 4.09-4.18 (m, 1 H), 5.51-4.62 (m, 2 H), 6.33 (d, 1 H), 6.78 (t, 1 H), 7.00 (bs, 1 H), 7.18 (d, 1 H), 7.25-7.33 (m, 4 H), 7.49-7.61 (m, 5 H), 7.98 (s, 1 H), 8.29-8.36 (m, 2 H), 8.79 (d, 1 H), 11.31 (s, 1 H), 11.75 (s, 1 H).

### D.8.) 2-(2-Amino-pyrimidin-4-yl)-1H-indole-5-carboxylic acid {1-carbamoyl-2-[(phenyl)-pyridin-2-yl-amino]-ethyl}-amide (45)

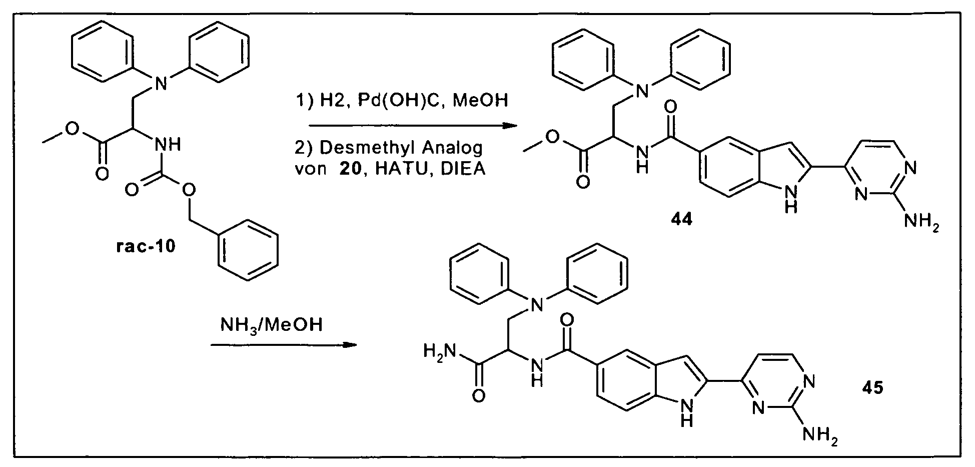

### D.8.1.) 3-[(Phenyl)-pyridin-2-yl-amino]-2-{[2-(2-amino-pyrimidin-4-yl)-1H-indole-5-carbonyl]-amino}-propionic acid methyl ester (44)

Aus 540 mg **rac-10** wurden unter analoger Durchführung wie unter B.1.4) und D.1.1.) beschrieben, 816 mg (80 %) des Methylesters **44** erhalten. Summenformel C₂₉H₂₆N₆O₃; M.W. = 506.56; MS (M+H) 507.37.

### D.8.2.) 2-(2-amino-pyrimidin-4-yl)-1H-indole-5-carboxylic acid {1-carbamoyl-2-[(phenyl)-pyridin-2-yl-amino]-ethyl}-amide (45)

Aus 150 mg **44** wurden unter analoger Durchführung wie unter D.2.2.) beschrieben, 162 mg (67 %) des Amids **45** erhalten.
Summenformel C₂₈H₂₅N₇O₂; M.W. = 491.56; MS (M+H) 492.32.
¹H NMR (DMSO-d₆) 3.18 (s(b), 3H), 4.05 - 4.13 (m, 2H), 4.85 (q,1H), 6.58 (s(b), 2H), 6.88 - 7.59 (m, 19H), 7.98 (s, 1H), 8.25 (d, J = 3 Hz, 1H), 8.35 (d, J = 2 Hz, 1H), 11.78 (s, 1H).

### D.9.) 2-(2-Methylamino-pyrimidin-4-yl)-1H-indole-5-carboxylic acid {1-carbamoyl-2-[(-phenyl)-pyrimidyl-2-yl-amino]-ethyl}-amide (47)

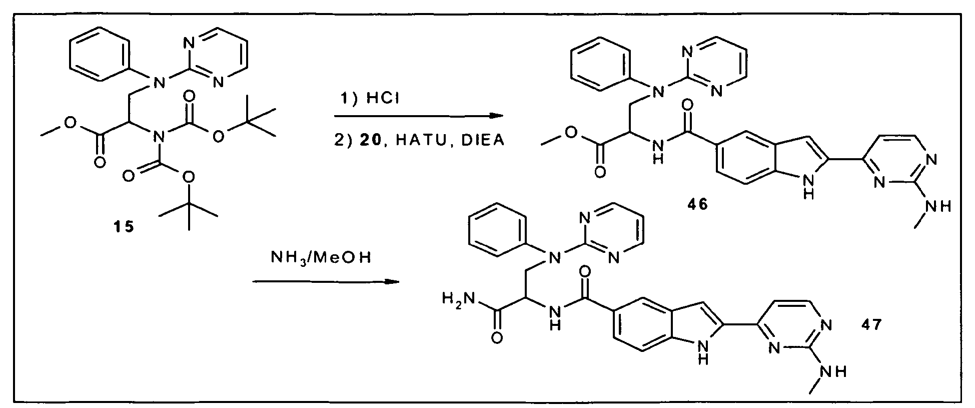

### D.9.1.) 3-[(phenyl)-pyridimyl-2-yl-amino]-2-{[2-(2-methylamino-pyrimidin-4-yl)-1H-indole- 5-carbonyl]-amino}-propionic acid methyl ester (46)

Aus 2.36 g **15** wurden unter analoger Durchführung wie unter D.2.1.) beschrieben, 1.75 mg (67 %) des Methylesters **46** erhalten.
Summenformel C₂₈H₂₆N₈O₃; M.W. = 522.57; MS (M+H) 523.3.

### D.9.2.) 2-(2-Methylamino-pyrimidin-4-yl)-1H-indole-5-carboxylic acid {1-carbamoyl-2-[(phenyl)-pyrimidyl-2-yl-amino]-ethyl}-amide (47)

Aus 700 mg **46** wurden unter analoger Durchführung wie unter D.2.2.) beschrieben, 440 mg (65 %) des Amids **47** erhalten.
Summenformel C₂₇H₂₅N₉O₂; M.W. = 507.21; MS (M+H) 508.4.
¹H NMR (DMSO-d₆) 3.0 (s(b), 3H), 4.20-4.32 (m, 1 H), 4.45-4.59 (m, 2 H), 4.75 - 4.90 (m, 1 H), 6.75 (m, 1 H), 7.10-7.60 (m, 12 H), 7.95 (s, 1 H), -8.35 - 8.45 (m, 4 H), 11.85 (s(b), 1 H).

### D.10.) 2-(2-Methylamino-pyrimidin-4-yl)-1H-indole-5-carboxylic acid [1-(2-hydroxy- ethylcarbamoyl)-2-(phenyl-pyrimidin-2-yl-amino)-ethyl]-amide (49)

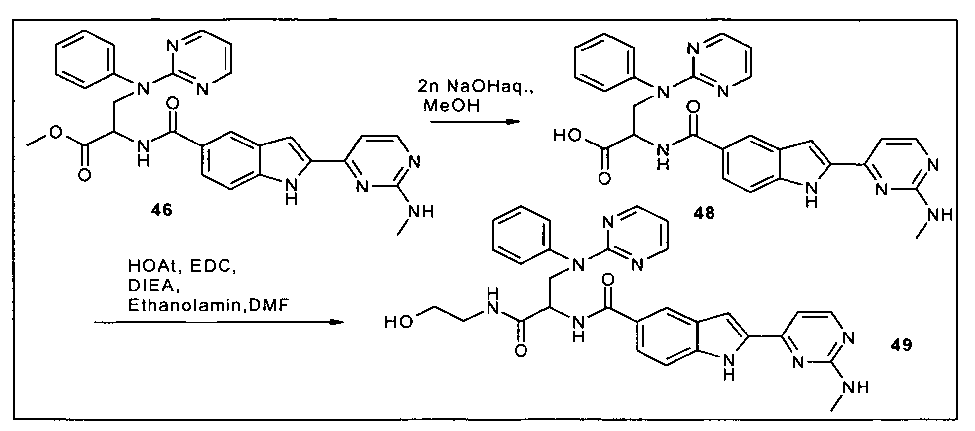

### D.10.1.) 2-{[2-(2-Methylamino-pyrimidin-4-yl)-1H-indole-5-carbonyl]-amino}-3-(phenyl-pyrimidin-2-yl-amino)-propionic acid (48)

4.0 g des Methylesters **46** wurde in 400 ml Methanol gelöst. Dazu wurden 40 ml 2 N wässriger NaOH Lösung gegeben und 12 h bei Raumtemperatur gerührt. Nach dem Evaporieren der Lösungsmittel wurde der Rückstand mit Wasser aufgelöst und mit gesättigter NaH₂PO₄-Lsg auf pH~5 eingestellt. Der entstandene Niederschlag wurde abfiltriert und mit Wasser gewaschen. Man erhielt so 1.3 g (Ausbeute 93%) die Säure **48**.
Summenformel C₂₉H₂₆N₆O₃; M.W. = 506.21; MS (M+H) 507.3. D.10.2.) 2-(2-Methylamino-pyrimidin-4-yl)-1H-indole-5-carboxylic acid [1-(2-hydroxy- ethylcarbamoyl)-2-(phenyl-pyrimidin-2-yl-amino)-ethyl]-amide (**49**) 200 mg der Säure **48** wurden in 2 ml absoluten DMF gelöst. Dazu gab man 94 mg HOAt und 158 µl DIEA. Anschließend wurden 56 µl Ethanolamine zugetropft auf 0°C abgekühlt und 195 mg EDC zugegeben. Nach 2 Tagen Rühren bei Raumtemperatur wurde das Lösungsmittel evaporiert und das Rohprodukt mittels MPLC (Elutionsmittel: DCM : MeOH = 9:1) gereinigt. Ausbeute: 108 mg (50%) des Titelamids **49**. Summenformel C₃₁H₃₁N₈O₂; M.W. = 549.64; MS (M+H) 550.4.
¹H NMR (DMSO-d₆) 1,2 (t, 2H), 3.0 (s(b), 3H), 3,35 (t, 1H), 4.00-4.32 (m, 2H), 4.80 - 4.99 (m, 1 H), 6.95 (m, 1 H), 7.00-7.65 (m, 7 H), 7.90 (m, 1 H), -8.35 - 8.40 (m, 1 H), 11.90 (s(b), 1 H).

### D.11.) (S)-2-{[2-(2-Methylamino-pyrimidin-4-yl)-1H-indole-5-carbonyl]-amino}-3-[phenyl-(4-trifluoromethyl-pyrimidin-2-yl)-amino]-propionic acid (54)

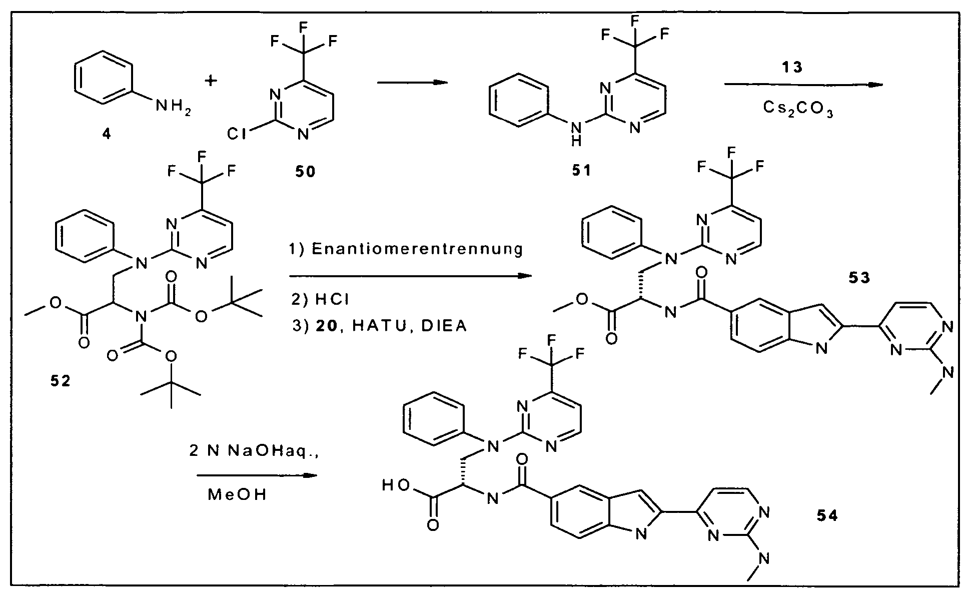

### D.11.1.) Phenyl-(4-trifluoromethyl-pyrimidin-2-yl)-amine (51)

Aus 5.1 g Anilin (**4**) und 5 g Chlorpyrimidin **50** wurden unter analoger Durchführung wie unter A.1.) beschrieben 5.1 g (78 %) Anilin **51** erhalten.
Summenformel C₁₁H₈F₃N₃; M.W. = 239.20; MS (M+H) 240.1.

### D.11.2.) (3-(N-Phenyl-N-4-trifluormethylpyrimidin-2-yl)-amino)-2-di-tert.-butyloxycarbonyl)-amino-propionsäure methylester (52)

Aus 2.5 g (8.4 mmol) Acrylat **13**, 3 g (12.5 mmol) Anilin **51** und 16 g (50 mmol) Cäsiumcarbonat wurden analog der Durchführung wie unter D.3.1.) beschrieben, 3.9 g (86 %) des Esters **52** erhalten. Summenformel C₂₅H₃₁F₃N₄O₆;
M.W. = 540.54; MS (M+H) 541.2.

### D.11.3.) Die Enantiomerentrennung erfolgte wie unter B.2.1.) beschrieben.

### D.11.4.) S-2-{[2-(2-Methylamino-pyrimidin-4-yl)-1H-indole-5-carbonyl]-amino}-3-[phenyl-(4-trifluoromethyl-pyrimidin-2-yl)-amino]-propionic acid methyl ester (53)

Aus 743 mg (1.375 mmol) des S-Enantiomers des Esters **52** und 550 mg (1.436 mmol) der Säure **20** wurden analog der Durchführung wie unter D.3.3. beschrieben, 467 mg (58 %) **53** erhalten.
Summenformel C₂₉H₂₅F₃N₈O₃; M.W. = 590.57; MS (M+H) 591.7.

### D.11.5.) (S)-2-{[2-(2-Methylamino-pyrimidin-4-yl)-1H-indole-5-carbonyl]-amino}-3-[phenyl-(4-trifluoromethyl-pyrimidin-2-yl)-amino]-propionsäure (54)

Aus 97 mg (0.164 mmol) des Esters **53** wurden analog der Durchführung wie unter D.10.1.) beschrieben, 38 mg (40 %) der Säure **54** erhalten.
Summenformel C₂₈H₂₃F₃N₈O₃; M.W. = 576.54; MS (M+H) 577.7.
¹H NMR (DMSO-d₆) 2.95 (s, 3 H), .4.27-4.34 (m, 1 H), 4.54-4.63 (m, 1 H), 4.83-4.92 (m, 1 H), 6.90 (bs, 1 H), 7.15 (d, 2 H), 7.19-7.23 (m, 1 H), 7.27-7.36 (m, 5 H), 7.45-7.55 (m, 2 H), 7.96 (s, 1 H), 8.32 (s, 1 H), 8.41 (bs, 1 H), 8.66 (d, 1 H), 11.70 (s, 1 H).

### D.12.) 2-(2-Methylamino-pyrimidin-4-yl)-1H-indole-5-carboxylic acid {1-carbamoyl-2-[(4-fluoro-phenyl)-(5-methyl-pyrimidin-2-yl)-amino]-ethyl}-amide (61)

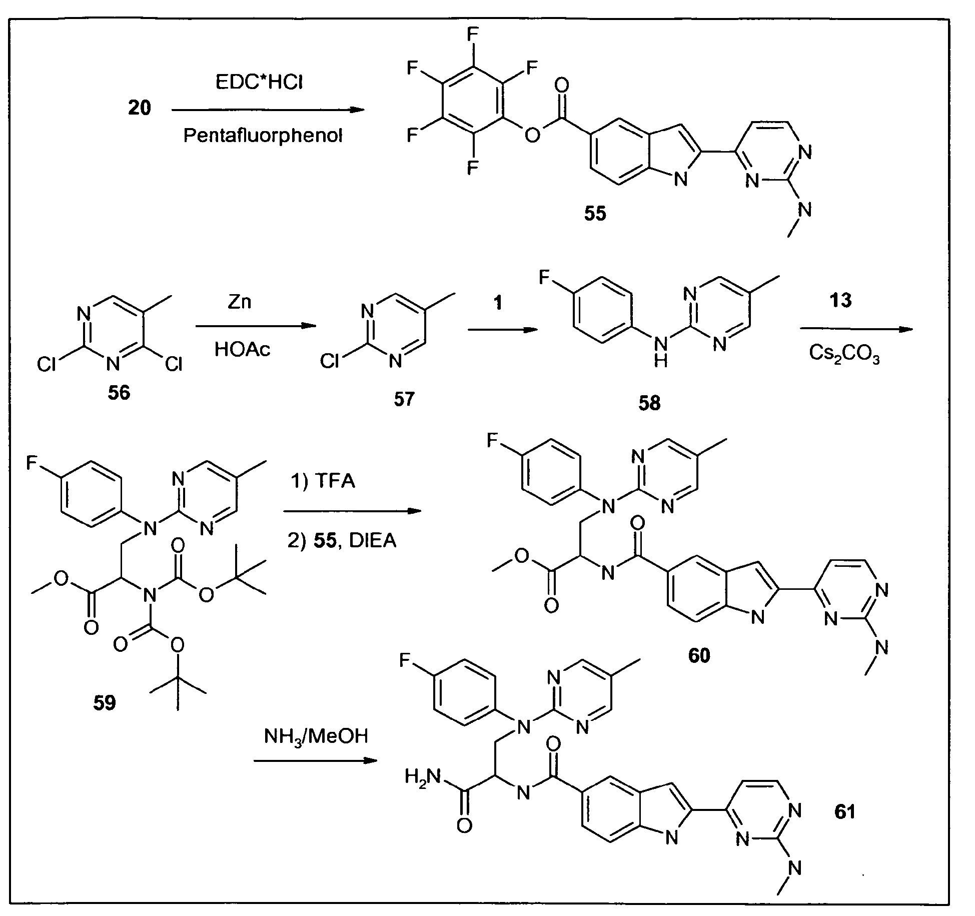

### D.12.1.) 2-(2-Methylamino-pyrimidin-4-yl)-1H-indole-5-carboxylic acid pentafluorophenyl ester (55)

6.38 g (23.78 mmol) Säure **20** wurden in 100 ml THF suspendiert. Dazu gab man 5.25 g (28.54 mmol) Pentafluorphenol und 5.47 g (28.54 mmol) 1-Ethyl-3-(3-dimethylaminopropyl)-carbodiimid Hydrochlorid (EDC*HCl). Man ließ 15 h bei RT rühren, entfernte das Lösungsmittel i. V. und verteilte den Rückstand zwischen 300 ml ges. NaHCO₃-Lösung und 300 ml Essigester. Die Feststoffe wurden über Kieselgur abfiltriert und der Rückstand 2 mal mit je 100 ml Essigester gewaschen. Die Phasen wurden getrennt und die wässrige Phase 2 mal mit je 100 ml Essigester extrahiert. Die vereinigten organischen Phasen wurden mit 200 ml ges. NaCl-Lösung gewaschen und dann mit MgSO₄ getrocknet. Nach Entfernen der Lösungsmittel i. V. wurde der Rückstand an Kieselgel mit Heptan/Essigester 1:1 chromatographiert. Man erhielt 4.7 g (46 %) des Pentafluorphenylesters **55**. Summenformel C₂₀H₁₁F₅N₄O₂; M.W. = 434.33; MS (M+H) 435.4.

### D.12.2.) 2-Chloro-5-methyl-pyrimidine (57)

10.0 g (61.35 mmol) 2,4-Dichlor-5-methylpyrimidin (**56**) wurden in 50 ml THF gelöst. Man gab 12.93 g (184 mmol) Zink hinzu und erhitzte zum Rückfluss. Dann wurde langsam eine Lösung von 3.51 ml (61.35 mmol) Essigsäure in 10 ml THF zugetropft. Nach Beendigung der Zugabe wurde noch 1 h zum Rückfluss erhitzt. Man tropfte weitere 1.5 ml Essigsäure in 5 ml THF zu und erhitzte 1 h zum Rückfluss. Man ließ dann auf RT abkühlen, filtrierte über Kieselgur und wusch 2 mal mit je 20 ml THF nach. Die Lösungsmittel wurden i. V. entfernt und der Rückstand an Kieselgel chromatographiert. Man erhielt 4.7 g (60 %) des Chlorpyrimidins **57**.
Summenformel C₅H₅ClN₂; M.W. = 128.56; MS (M+H) 129.2.

### D.12.3.) (4-Fluoro-phenyl)-(5-methyl-pyrimidin-2-yl)-amine (58)

Aus 2.5 g (19.45 mmol) 2-Chlor-5-methylpyrimidin (**57**) und 2.7 g (24.31 mmol) 4-Fluoranilin (**1**) wurden unter analoger Durchführung wie unter A.1.) beschrieben 1.8 g (45 %) Anilin **58** erhalten. Summenformel C₁₁H₁₀FN₃; M.W. = 203.22; MS (M+H) 204.2.

### D.12.4.) (3-(N-4-Fluor-phenyl-N-5-methyl-pyrimidin-2-yl)-amino)-2-di-tert.-butyloxycarbonyl)-amino-propionsäure methylester (59)

Aus 2.67 g (8.86 mmol) Acrylat **10,** 1.8 g (8.86 mmol) Anilin **58** und 8.66 g (26.58 mmol) Cäsiumcarbonat wurden analog der Durchführung wie unter D.3.1.) beschrieben, 2.88 g (64 %)des Esters **59** erhalten.
Summenformel C₂₅H₃₃FN₄O₆; M.W. = 504.56; MS (M+H) 505.6.

### D.12.5.) 3-[(4-Fluoro-phenyl)-(5-methyl-pyrimidin-2-yl)-amino]-2-{[2-(2-methylamino-pyrimidin-4-yl)-1H-indole-5-carbonyl]-amino}-propionic acid methyl ester (60)

500 mg (0.991 mmol) des Esters **59** wurden in 10 ml Dichlormethan gelöst und auf 0 °C gekühlt. Man gab 5 ml TFA hinzu, ließ auf RT kommen und 1 h nachrühren. Die Lösungsmittel wurden i. V. entfernt. Der Rückstand wurde in 10 ml DMF aufgenommen und 430 mg (0.991 mmol) **55** sowie 1.38 ml (7.93 mmol) DIEA zugegeben. Man ließ 15 h bei RT rühren, entfernte die Lösungsmittel i. V. und chromatographierte den Rückstand an Kieselgel mit Heptan/Essigester 1:3. Man erhielt 423 mg (77 %) **60.** Summenformel C₂₉H₂₇FN₈O₃; M.W. = 554.59; MS (M+H) 555.2.

### D.12.6.) 2-(2-Methylamino-pyrimidin-4-yl)-1H-indole-5-carboxylic acid {1-carbamoyl-2-[(4-fluoro-phenyl)-(5-methyl-pyrimidin-2-yl)-amino]-ethyl}-amide (61)

Aus 260 mg (0.469 mmol) Ester **60** wurden unter analoger Durchführung wie unter D.2.2.) beschrieben, 250 mg (99 %) Amid **61** erhalten.
Summenformel C₂₈H₂₆FN₉O₂; M.W. = 539.58; MS (M+H) 540.2.
¹H NMR (DMSO-d₆) 2.11 (s, 3 H), 2.95 (s, 3 H), 4.21 (dd, 1 H), 4.48 (dd, 1 H), 4.75-4.80 (m, 1 H), 7.01 (bs, 1 H), 7.10-7.16 (m, 4 H), 7.22-7.30 (m, 3 H), 7.43 (s, 1 H), 7.47-7.53 (m, 2 H), 7.91 (s, 1 H), 8.26 (s, 2 H), 8.29.8.34 (m, 2 H), 11.70 (s, 1 H).

### F.) Benzimidazol Endprodukte

### F.1.) 2-(2-Methylamino-pyrimidin-4-yl)-1H-benzoimidazol-5-carbonsäure-((S)1-carbamoyl-2-di phenylamino-ethyl)-amid (63)

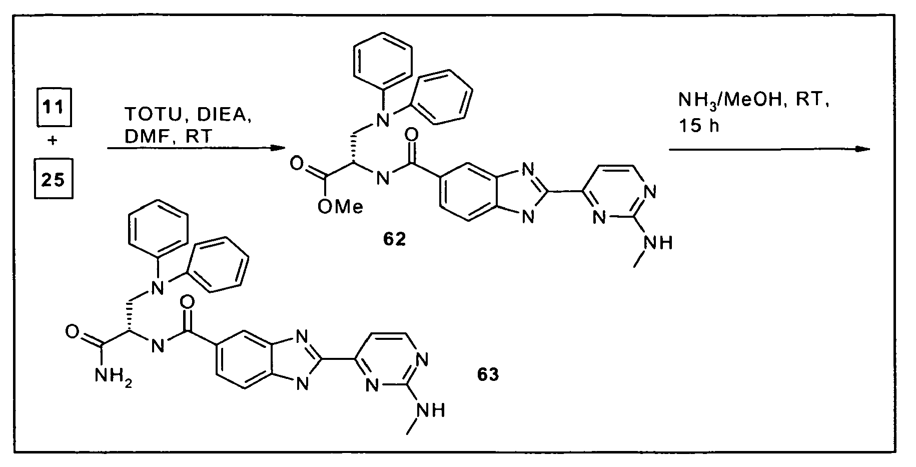

### F.1.1.) 3-Diphenylamino-2-{[2-(2-methylomino-pyrimidin-4-yl)-1H-benzimidazol-5-carbonyl]-(S)-amino}-propionsäure-methyl-ester(62)

2.6 g (9.6 mmol) 2-(2-Methylamin-pyrimidin-4-yl)-1H-benzimidazol-5-carbonsäure (**25**) wurden in 300 ml DMF gelöst und nacheinander mit 3.17 g (9.6 mmol) TOTU und 1.6 ml (11.6 mmol) Ethyldiisopropylamin versetzt. Man rührte 20 min bei 5°C und gab zu der Lösung 2.6 g (9.6 mmol) (S)-2-Amino-3-diphenylamino-propionsäure-methyl-ester (**11**) hinzu. Nach 16 h Rühren engte man unter vermindertem Druck ein, anschließend wurde der Methylester **62** durch Flash-Chromatographie an Kieselgel (DCM:MeOH= 9:1) isoliert. Ausbeute: 1.61 g (32%)
Summenformel C₂₉H₂₇N₇O₃; M.W. = 521.58; MS (M+H) 522.3.
¹H NMR (DMSO-d₆) 2.95 (s(b), 3H), 3.60 (s, 3H), 4.19 - 4.40 (m, 2H), 4.90 (q,1H), 6.90 - 7.10 (m, 6H), 7.25 - 7.35 (m, 6H), 7.40 (d, J = 4.5 Hz, 1H), 7.60 - 7.80 (d (b) 1H), 8.05 - 8.25 (d(b), 1H), 8.45 (d, J = 3 Hz, 1H), 8.90 (s(b), 1H), 11.85 (s(b), 1H).

### F.1.2.) 2-(2-Methylamino-pyrimidin-4-yl)-1H-benzoimidazol-5-carbonsäure ((S)1-carbamoyl-2- diphenylamino-ethyl)-amid (63)

50 ml Methanol (absolut) wurden bei 0°C mit Ammoniak gesättigt. Dazu wurde 0.5 g (0.959 mmol) 3-Diphenylamin-2-{[2-(2-methylamin-pyrimidin-4-yl)-1H-benzimidazol-5-carbonyl]-(S)-amin}- propionsäure-methyl-ester (**62**) gegeben und 24 h bei Raumtemperatur gerührt. Nach Evaporation des Lösungsmittels und überschüssigen Ammoniaks wurde durch Flash-Chromatographie an Kieselgel (DCM:MeOH= 19:1) das Amid **63** isoliert. Ausbeute: 0.43 g (89%)
Summenformel C₂₉H₂₈N₈O₂; M.W. = 506.57; MS (M+H) 507.2.
¹H NMR (DMSO-d₆) 2.95 (s(b), 3H), 4.02 - 4.35 (m, 2H), 4.85 (q,1H), 6.80 - 7.10 (m, 6H), 7.15 - 7.25 (m, 5H), 7.40 (d, J = 4.5 Hz, 1H), 7.58 (s(b), 1H), 7.68 (s(b), 1H), 8.06 - 8.19 (d(b), 1H), 8.40 - 8.58 (m, 2H), 13.10 (s, 1H).

### F.2.) 2-(2-Methylamino-pyrimidin-4-yl)-1H-benzimidazole-5-carboxylic acid {1-carbamoyl-2-[(- phenyl)-pyrimidin-2-yl-amino]-ethyl}-amide (65)

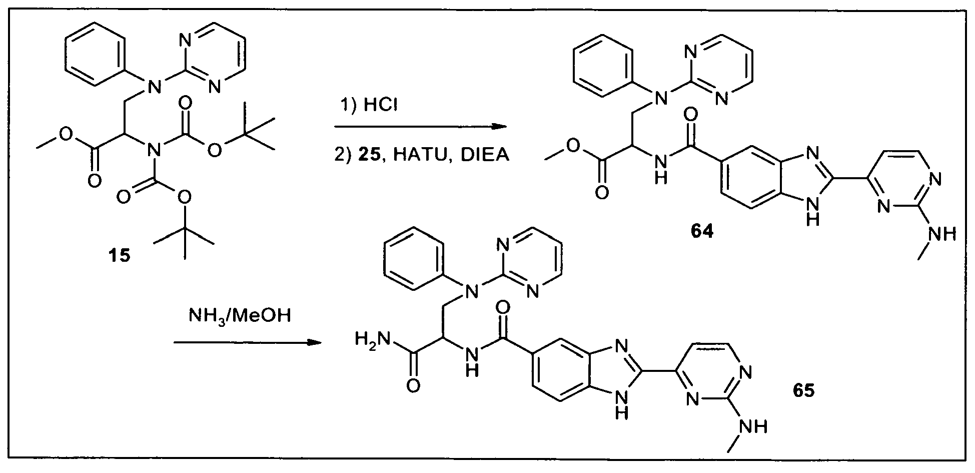

### F.2.1.) 3-[(phenyl)-pyridimidin-2-yl-amino]-2-{[2-(2-methylamino-pyrimidin-4-yl)-1H-benzimidazole- 5-carbonyl]-amino}-propionic acid methyl ester (64)

Aus 657mg **15** wurden unter analoger Durchführung wie unter D.2.1.) beschrieben, 210 mg (29 %) des Methylesters **64** erhalten. Summenformel C₂₇H₂₅N₉O₃; M.W. = 523.56; MS (M+H) 524.2.

### F.2.2.) 2-(2-Methylamino-pyrimidin-4-yl)-1H-benzimidazole-5-carboxylic acid {1-carbamoyl-2-[(phenyl)-pyrimidin-2-yl-amino]-ethyl}-amide (65)

Aus 200 mg **64** wurden unter analoger Durchführung wie unter D.2.2.) beschrieben, 110 mg (65 %) des Amids **65** erhalten. Summenformel C₂₆H₂₄FN₁₀O₂; M.W. = 508.55; MS (M+H) 509.3.
¹H NMR (DMSO-d₆) 3.0 (s(b), 3H), 4.20-4.32 (m, 1 H), 4.41-4.55 (m, 2 H), 4.80 - 4.90 (m, 1 H), 6.75 (m, 1 H), 7.10-7.50 (m, 10 H), 7.65 (q, 2 H), 8.10 (s, 1 H), -8.45 (d, 2 H), 8.50 (d, 1 H), 8.58 (d, 1 H), 12.95 (s(b), 1 H).

### F.3.) 2-(2-Methylamino-pyrimidin-4-yl)-1H-benzimidazole-5-carboxylic acid {1-carbamoyl-2-[(- phenyl)-pyridyl-2-yl-amino]-ethyl}-amide (67)

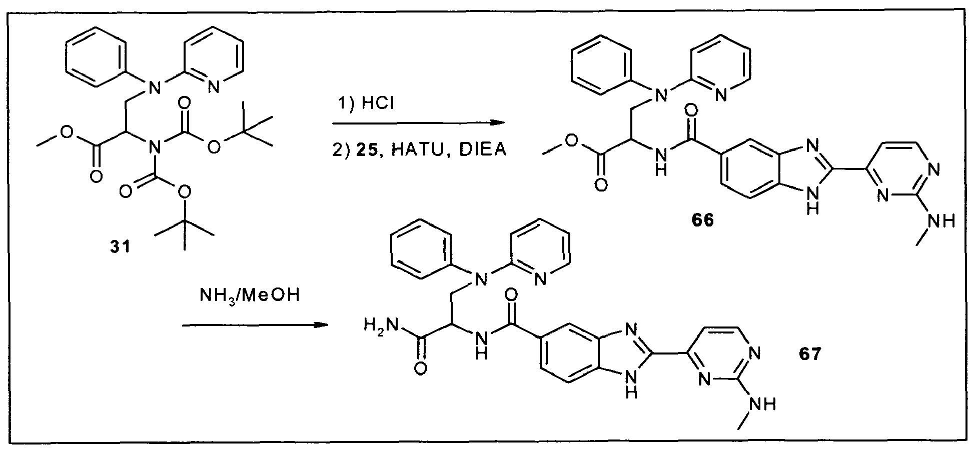

### F.3.1.) 3-[(Phenyl)-pyridyl-2-yl-amino]-2-{[2-(2-methylamino-pyrimidin-4-yl)-1H-benzimidazole- 5-carbonyl]-amino}-propionic acid methyl ester (66)

Aus 3.44 g **31** wurden unter analoger Durchführung wie unter D.2.1.) beschrieben, 0.85 g (22 %) des Methylesters **66** erhalten.
Summenformel C₂₈H₂₆N₈O₃; M.W. = 522.57; MS (M+H) 523.3.

### F.3.2.) 2-(2-Methylamino-pyrimidin-4-yl)-1H-benzimidazole-5-carboxylic acid {1-carbamoyl-2-[(phenyl)-pyridyl-2-yl-amino]-ethyl}-amide (67)

Aus 200 mg **66** wurden unter analoger Durchführung, wie unter D.2.2.) beschrieben, 160 mg (98 %) des Amids **67** erhalten.
Summenformel C₂₇H₂₅N₉O₂; M.W. = 507.56; MS (M+HCOO-) 552.3.
¹H NMR (DMSO-d₆) 3.0 (s(b), 3H), 4.20-4.32 (m, 1 H), 4.41-4.55 (m, 2 H), 4.70 - 4.80 (m, 1 H), 6.63 (m, 1 H), 6.85 (m, 1 H), 7.20-7.75 (m, 14 H), 8.10 (s, 1 H), -8.20 (d, 2 H), 8.50 (d, 1 H), 8.88 (d, 1 H).

### Pharmakologische Beispiele

### IκB-Kinase ELISA:

Die Aktivität der IκB-Kinase wurde mit einem ELISA, bestehend aus einem biotiniliertem Substratpeptid, welches die Aminosäuresequenz im Protein IκB von Serine 32 bis 36 enthält, und einem spezifischen poly- oder monoklonalen Antikörper (z.B. von New England Biolabs, Beverly, MA, USA, Kat.: 9240), der nur an die phosphorylierte Form des Peptids IκB bindet, bestimmt. Dieser Komplex wurde an einer antikörperbindenden Platte (Protein A beschichtet) immobilisiert und mit einem Konjugat aus einem biotinbindendem Protein und HRP (z.B. Streptavidin-HRP) detektiert. Die Aktivität wurde an Hand einer Standardkurve mit Substratphosphopeptid quantifiziert.

### Durchführung:

Zur Gewinnung des Kinasekomplexes wurden 10 ml HeLa S3-Zellextrakt S100 mit 40 ml 50mM HEPES, pH 7,5, verdünnt, auf 40% Ammoniumsulfat gebracht und auf Eis 30 Minuten inkubiert. Das präzipitierte Pellet wurde in 5 ml SEC Puffer (50 mm HEPES, pH 7,5, 1 mm DTT, 0,5 mm EDTA, 10 mm 2-Glyzerophosphat) gelöst, bei 20,000 x g für 15 Minuten zentrifugiert und durch einen 0,22 µm Filter filtriert. Die Probe wurde auf eine 320 ml Superose-6 FPLC Säule (Amersham Pharmacia Biotech AB, Uppsala, Schweden) aufgetragen, die mit SEC Puffer äquilibriert war und mit einer Flussrate von
2 ml/min bei 4 °C betrieben wurde. Die Fraktionen, die bei der Laufzeit des 670 kDa Molekulargewichtstandards lagen, wurden für die Aktivierung vereinigt . Die Aktivierung wurde durch eine 45-minütige Inkubation mit 100 nM MEKK1Δ, 250 µM MgATP, 10 mm MgCl₂.5 mm Dithiothreitol (DTT), 10 mm 2-Glyzerophosphat, 2,5 µM Microcystin-LR bei 37 °C erreicht. Das aktivierte Enzym wurde bei -80 °C gelagert.
Die in DMSO gelösten Testsubstanzen (2 µl) wurden 30 Minuten bei 25°C mit 43 µl aktiviertem Enzym (1:25 verdünnt in Reaktionspuffer 50 mm HEPES, pH 7,5, 10 mm MgCl₂, 5 mm DTT, 10 mm β-Glycerophosphat, 2,5 µM Microcystin-LR) vorinkubiert. Dann wurden 5 µl Substratpeptid (Biotin-(CH₂)₆-DRHDSGLDSMKD-CONH₂) (200 µM) dazugegeben, eine Stunde inkubiert und mit 150 µl 50 mm HEPES pH 7,5, 0.1% BSA, 50 mm EDTA, Antikörper [1:200] abgestoppt. 100 µl des abgestoppten Reaktionsgemisches bzw. einer Standardphosphopeptidverdünnungsreihe (Biotin-(CH₂)₆-DRHDS[PO₃]GLDSMKD-CONH₂) wurden dann in eine Protein-A Platte (Pierce Chemical Co., Rockford, IL, USA) überführt und 2 Stunden unter Schütteln inkubiert. Nach 3 Waschschritten mit PBS, wurden 100 µl 0,5 µg/ml Streptavidin-HRP (horseradish peroxidase) (verdünnt in 50 mm HEPES/ 0,1% BSA) für 30 Minuten hinzugegeben. Nach 5 Waschschritten mit PBS, wurden 100 µL TMB-Substrat (Kirkegaard & Perry Laboratories, Gaithersburg, MD, USA) hinzugegeben und die Farbentwicklung durch Zugabe von 100 µL 0,18 M Schwefelsäure abgestoppt. Die Absorption wurde bei 450 nm gemessen. Die Standardkurve wurde durch lineare Regression entsprechend einer 4-Parameter Dosis-Wirkungsbeziehung erzeugt. An Hand dieser Standardkurve wurde die Enzymaktivität bzw. deren Inhibition durch die Testsubstanzen quantifiziert.

Die IC₅₀ für 2-(2-Methylamin-pyrimidin-4-yl)-1H-indol-5-carbonsäure [(S)-2-diphenylamin-1-(5-oxo- 4,5-dihydro-[1,3,4]oxadiazol-2-yl)-ethyl]-amid betrug 0,050 µM.

### Blutplasmaspiegel von 2-(2-Methylamin-pyrimidin-4-yl)-1H-indol-5-carbonsäure [(S)-2-diphenylamin-1-(5-oxo- 4,5-dihydro-[1,3,4]oxadiazol-2-yl)-ethyl]-amid

Die Verbindung 2-(2-Methylamin-pyrimidin-4-yl)-1H-indol-5-carbonsäure [(S)-2-diphenylamin-1-(5-oxo-4,5-dihydro-[1,3,4]oxadiazol-2-yl)-ethyl]-amid, im folgenden Verbindung 28 genannt, wurde männlichen C57/BL6-Mäusen verabreicht. Dazu wurden jeweils etwa 25 mg der Verbindung 28 pro kg Körpergewicht der Mäuse nassgemahlen in 0,5 % Hydroxyethylcellulose (HEC) als Suspension oral (über Schlundsonde) gegeben. Das Ziehen der Blutproben erfolgte nach 0,25; 0,5; 1; 2; 4; 6 und 8 Stunden (zu jedem der genannten Zeitpunkte wurde Tötungsblut von jeweils 2 Tieren entnommen). Die Blutproben wurden in Heparin-Plasma umgewandelt. Die Plasmaproben wurden bis zur Analyse bei -20°C gelagert. Analytik:
Die Plasmaproben wurden aufgetaut. Anschließend wurden die für die Analytik störenden Plasmaproteine mittels Acetonitril augegefällt.
Aufarbeitung: 50 µl Plasma + 20 µl interner Standard (5µg/ml) + 50 µl Puffer (2 mmol Ammoniumformiatlösung, pH 2,6/Acetonitril, 40:60, v/v) wurden etwa 10 sec auf einem Whirlmixer gemischt. Anschließend wurde 150 µl Acetonitril zugefügt und erneut etwa 10 sec gemischt. Die Proben wurden anschließend zentrifugiert (Hettich, EBA 12, etwa 12000 Umdrehungen pro Minute). Der Überstand (etwa 200 µl) wurde in Glasgefäße transferiert. 70 µl des Überstandes wurden injiziert.
Aus dem jeweiligen Überstand wurde der Plasmaspiegelgehalt der Verbindung 13 mittels LC-MS/MS nach der folgenden Methode bestimmt:
HPLC-System: Agilent 1100
Software: Analyst
Säule: 125x4mm Nucleosil 120 5 C18 (Machery&Nagel)
Säulenlänge: 125 mm
Detektion: LC-MS/MS
MS-Gerät: PE - Sciex API 365 (Triple Quadrupole Massespektrometer)
Software: MacQuan Software (PE-Sciex)
Detektionsart: MS/MS (MRM)
Flußrate: 0,5 mL/min
Injektionsvolumen: 70 µl
Interner Standard: SK-7 in Acetonitril
Mobile Phase: Acetonitril/2 mMol Ammoniumformiatlösung, pH 2,6 (70:30, v/v) Retentionszeiten (Rt):
   Interner Standard: 4,4 min
   Verbindung 28: 3,9 min
   Die untere Nachweisgrenze der Methode liegt bei 0,01 µg/mL.

### Ergebnisse:

Der Plasmaspiegel der Verbindung 28 betrug maximal 4,3 µg/mL. Die Exposition gemessen als AUC = Area under the curve betrug 5,4 µg/mLxh..

### Proteintyrosinkinase

Als Beispiele für die Spezifität der gefundenen IkB-Kinase-Inhibitoren wurde der IC₅₀-Wert bei der Kinase-Enzym Proteintyrosinkinase bestimmt.
Die Proteintyrosinkinase Aktivität wurde mit dem entsprechenden Testkit von Upstate Biotechnologie gemäß der Vorschrift des Hersteller bei einer ATP-Konzentration von 50 µM bestimmt. Abweichend wurden statt Phosphocellulosefilter Multi-Screen-Platten (Millipore; Phosphocellulose MS-PH, Kat. MAPHNOB10, oder Durapore PVDF , Kat . MADVN0B 50) mit dem entsprechenden Absaugsystem verwendet. Alts Testkit-Substrat wurde Poly (Glu, Tyr 4:1) (Sigma Kat. P0275) Testkonzentration 1 mg/ml, eingesetzt. Die Platten wurden anschließend in einem Wallac MicroBeta Szintillationszähler vermessen. Es wurde jeweils 100 µM der Testsubstanz verwendet.
Die Testsubstanz wurde in Doppelbestimmung getestet. Die IC₅₀-Berechnungen wurden mit dem Softwarepaket GraFit 3.0 durchgeführt.

Die IC₅₀ für 2-(2-Methylamin-pyrimidin-4-yl)-1H-indol-5-carbonsäure [(S)-2-diphenylamin-1-(5-oxo- 4,5-dihydro-[1,3,4]oxadiazol-2-yl)-ethyl]-amid (Verbindung 28) betrug im Proteintyrosinkinaseassay 82,5 µM.Vergleichsversuch: Die Verbindung 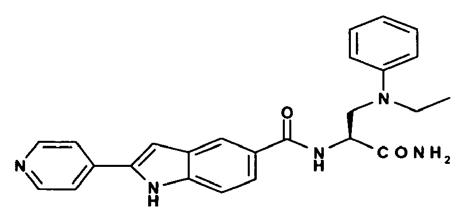 wurde wie in WO 01/30774 beschrieben hergestellt und wird im folgenden als Vergleichsverbindung bezeichnet. Die Vergleichsverbindung wurde männlichen NMRI-Mäusen verabreicht. Dazu wurden jeweils etwa 50 mg der Vergleichsverbindung pro kg Körpergewicht der Mäuse in 0,5 % HEC als Suspension oral (über Schlundsonde) gegeben. Das Ziehen der Blutproben erfolgte nach 0,25; 0,5; 1; 2; 4; 6 und 8 Stunden (zu jedem der genannten Zeitpunkte wurde Tötungsblut von jeweils 2 Tieren entnommen). Die Blutproben wurden in Heparin-Plasma umgewandelt. Die Plasmaproben wurden bis zur Analyse bei -20°C gelagert.
Analytik: Die Analytik wurde mit HPLC/UV durchgeführt.

Aufarbeitung: 50 µl Plasma + 20 µl interner Standard (5µg/ml) + 50 µl Puffer (1% Ameisensäure/Acetonitril, 40:60, v/v) wurden etwa 10 sec auf einem Whirlmixer gemischt. Anschließend wurde 150 µl Acetonitril zugefügt und erneut etwa 10 sec gemischt. Die Proben wurden anschließend zentrifugiert (Hettich, EBA 12, etwa 12000 Umdrehungen pro min).
Der Überstand (etwa 200 µl) wurde in Glasgefäße transferiert. 100 µl des Überstandes wurden injiziert.

Aus dem jeweiligen Überstand wurde der Plasmaspiegelgehalt der Vergleichsverbindung mittels HPLC/UV nach der folgenden Methode bestimmt:
HPLC-System: Gynkoteck P580 HPG-Pumpe + Gilson Abimed XL-231 Autosampler
Software: Mass-chrom
Säule: 125x4mm Protosil 120 3 ODS AQ 3 (Fa. Bischoff)
Säulenlänge: 125 mm
Detektion: LC-MS/MS
MS-Gerät: PE - Sciex API 365 (Triple Quadrupole Massespektrometer)
Software: MacQuan Software (PE-Sciex)
Detektionsart: MS/MS (MRM)
Flußrate: 0,5 mL/min
Injektionsvolumen: 100 µl
Interner Standard: SK-7 (Aventis-Verbindung) in Acetonitril
Mobile Phase: Acetonitril/2 mMol Ammoniumformiatlösung, pH 2,6 (70:30, v/v) Retentionszeiten (Rt):
   Interner Standard: 4 min
   Vergleichsverbindung: 1,5 min

Die untere Nachweisgrenze mit 0,01 µg/mL war identisch zu der mittels LC-MS/MS im Beispiel mit der Verbindung 28.

Ergebnisse: Der Plasmaspiegel der Vergleichsverbindung betrug maximal 1,5 µg/mL. Die Exposition gemessen als AUC = Area under the curve betrug 1,7 µg/mLxh.
Im Vergleich zum Beispiel mit der Verbindung 28 war der maximale Blutplasmaspiegel etwa
60 % niedriger im Vergleichsversuch, obwohl die Vergleichsverbindung mit 50 mg/kg doppelt so hoch dosiert wurde wie bei der Verbindung 28. Das gleiche Ergebnis zeigen auch die ermittelten AUC-Werte für die Vergleichsverbindung.

Der IC₅₀ für die Vergleichsverbindung betrug im oben beschriebenen Proteintyrosinkinaseassay 46,35 µM. Daher ist der IC₅₀ deutlich besser als für die Verbindung 28.
Noch deutlicher wird die Verbesserung der Spezifität in Bezug auf die IκB-Kinase, wenn man das Verhältnis der IC₅₀ Werte von Proteintyrosinkinase zu IκB-Kinase vergleicht. Dieser Quotient beträgt für die Verbindung 28 1650 (82,5/0,05) und für die Vergleichsverbindung 46,35 (46,35/1,0 ; gemäß den Daten aus WO 01/30774).

Analog wurde die Spezifität-Verhältnisse bzw. Plasmaspiegel und Exposition der weiteren Beispiele bestimmt

| Beispiel Nr. | Molekular Formel Neutralverbindung | Molekular Gewicht | IKK IC50 50µM | Spezifitäts-Verhältnis |
|---|---|---|---|---|
| 28 | C30H26N8O3 | 546,59 | 0,05 | 1650 |
| 30 | C28H25FN8O2 | 524,56 | 0,05 | > 200 |
| 33 | C29H25N9O3 | 547,58 | 0,012 | > 833 |
| 35 | C27H23FN8O2 | 510,54 | 0,01 | > 1000 |
| 36 | C29H25FN10O | 548,59 | 0,005 | > 2000 |
| 42 | C26H24N8O2S | 512,60 | 0,009 | > 1110 |
| 43 | C29H28N8O3 | 536,60 | 0,0008 | > 12500 |
| 45 | C28 H25 N7 O2 | 491,55 | 0,015 | > 665 |
| 47 | C27H25N9O2 | 507,56 | 0,006 | > 1665 |
| 49 | C31 H31 N7 O3 | 549,63 | 0,035 | > 285 |
| 54 | C28H23F3N8O3 | 576,54 | 0,003 | > 3330 |
| 61 | C28H26FN9O2 | 539,58 | 0,006 | > 1650 |
| 63 | C28 H26 N8 O2 | 506,57 | 0,003 | > 1000 |
| 65 | C26H24N10O2 | 508,55 | 0,004 | > 2500 |
| 67 | C27H25N9O2 | 507,56 | 0,002 | > 5000 |

| | | | | |
|---|---|---|---|---|
| > bedeutet besser als | | | | |

## Patentansprüche

1. Verbindung der Formel IV, 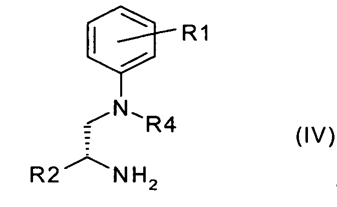 wobei,
R1 für
1. Wasserstoffatom,
2. F, CI, J oder Br,
3. -(C₁-C₄)-Alkyl,
4. -CN,
5. -CF₃,
6. -OR⁵, worin R⁵ für Wasserstoffatom oder -(C₁-C₄)-Alkyl steht,
7. -N(R⁵)-R⁶, worin R⁵ und R⁶ unabhängig voneinander für Wasserstoffatom oder
- (C₁-C₄)-Alkyl stehen,
8. -C(O)-R⁵, worin R⁵ für Wasserstoffatom oder -(C₁-C₄)-Alkyl steht, oder
9. -S(O)ₓ-R⁵, worin x die ganze Zahl Null, 1 oder 2 bedeutet und R⁵ Wasserstoffatom oder -(C₁-C₄)-Alkyl bedeutet, steht,
R2 für
1. einen Heteroarylrest aus der Gruppe 3-Hydroxypyrro-2,4- dion, Imidazol, Imidazolidin, Imidazolin, Indazol, Isothiazol, Isothiazolidin, Isoxazol, 2-Isoxazolidin, Isoxazolidin, Isoxazolon, Morpholin, Oxazol, 1,3,4-Oxadiazol, Oxadiazolidindion, Oxadiazolon, 1,2,3,5-Oxathiadiazol- 2-oxid, 5-Oxo-4,5-dihydro-[1,3,4]oxadiazol, 5-Oxo-1,2,4-thiadiazol, Piperazin, Pyrazin, Pyrazol, Pyrazolin, Pyrazolidin, Pyridazin, Pyrimidin, Tetrazol, Thiadiazol, Thiazol, Thiomorpholin, Triazol oder Triazolon, steht, und
der Heteroarylrest unsubstituiert ist oder ein-, zwei- oder dreifach unabhängig voneinander substituiert ist durch
1.1 -C(O)-R⁵, worin R⁵ für Wasserstoffatom oder - (C₁-C₄)-Alkyl steht,
1.2 -(C₁-C₄)-Alkyl,
1.3 -O-R⁵, worin R⁵ für Wasserstoffatom oder -(C₁-C₄)-Alkyl steht,
1.4 -N(R⁵)-R⁶, worin R⁵ und R⁶ unabhängig voneinander für Wasserstoffatom oder -(C₁-C₄)-Alkyl stehen,
1.5 Halogen oder
1.6 Keto-Rest,
2. -C(O)-R⁵, worin R⁵ für Wasserstoffatom oder -(C₁-C₄)-Alkyl steht,
3. C(O)-OR⁵, worin R⁵ für Wasserstoffatom oder -(C₁-C₄)-Alkyl steht, oder
4. -C(O)-N(R⁷)-R⁸, worin R⁷ und R⁸ unabhängig voneinander für Wasserstoffatom, -(C₁-C₄)-Alkyl-OH, -O-(C₁-C₄)-Alkyl oder - (C₁-C₄)-Alkyl stehen,
R4 für
1. einen Heteroarylrest aus der Gruppe Pyrrol, Furan, Thiophen, Imidazol, Pyrazol, Oxazol, Isoxazol, Thiazol, Isothiazol, Tetrazol, 1,2,3,5-Oxathiadiazol-2-Oxide, Triazolone, Oxadiazolon, Isoxazolon, Oxadiazolidindion, Triazol, 3-Hydroxypyrro-2,4-dione, 5-Oxo- 1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, β- Carbolin und benz-anellierte, cyclopenta-, oder cyclohexa- Derivate dieser Heteroarylreste,
wobei der Heteroarylrest unsubstituiert ist oder ein-, zwei- oder dreifach unabhängig voneinander substituiert ist durch -(C₁-C₅)-Alkyl, - (C₁-C₅)-Alkoxy, Halogen, Nitro, Amino, Trifluormethyl, Hydroxyl, Hydroxy-(C₁-C₄)-alkyl, Methylendioxy, Ethylendioxy, Formyl, Acetyl, Cyano, Hydroxycarbonyl, Aminocarbonyl oder -(C₁-C₄)-Alkoxycarbonyl, oder
2. einen Arylrest aus der Gruppe Phenyl, Naphthyl, 1-Naphthyl, 2- Naphthyl, Biphenylyl, 2-Biphenylyl, 3-Biphenylyl und 4-Biphenylyl, Anthryl oder Fluorenyl steht, und
der Arylrest unsubstituiert ist oder ein-, zwei- oder dreifach unabhängig voneinander substituiert ist durch -(C₁-C₅)-Alkyl, -(C₁-C₅)-Alkoxy, Halogen, Nitro, Amino, Trifluormethyl, Hydroxyl, Hydroxy-(C₁-C₄)-alkyl, Methylendioxy, Ethylendioxy, Formyl, Acetyl, Cyano, Hydroxycarbonyl, Aminocarbonyl oder -(C₁-C₄)-Alkoxycarbonyl.

2. Verbindung der Formel IV gemäß Anspruch 1, wobei
R1 für
1. Wasserstoffatom,
2. F, Cl, J oder Br,
3. -(C₁-C₄)-Alkyl,
4. -CN,
5. -CF₃,
6. -OR⁵, worin R⁵ für Wasserstoffatom oder -(C₁-C₄)-Alkyl steht,
7. -N(R⁵)-R⁶, worin R⁵ und R⁶ unabhängig voneinander für Wasserstoffatom oder
-(C₁-C₄)-Alkyl stehen,
8. -C(O)-R⁵, worin R⁵ für Wasserstoffatom oder -(C₁-C₄)-Alkyl steht, oder
9. -S(O)ₓ-R⁵, worin x die ganze Zahl Null, 1 oder 2 bedeutet und R⁵ Wasserstoffatom oder -(C₁-C₄)-Alkyl bedeutet, steht,
R2 für
1. einen Heteroarylrest aus der Gruppe Imidazol, Isothiazol, Isoxazol, 2-Isoxazolidin, Isoxazolidin, Isoxazolon, 1,3,4-Oxadiazol, Oxadiazolidindion, 1,2,3,5- Oxadiazolon, Oxazol, 5-Oxo-4,5-dihydro- [1,3,4]oxadiazol, Tetrazol, Thiadiazol, Thiazol, Triazol oder Triazolon steht, und
der Heteroarylrest unsubstituiert ist oder ein-, zwei- oder dreifach unabhängig voneinander substituiert ist durch
1.1 Keto-Rest,
1.2 F, Cl, J oder Br oder
1.3 -(C₁-C₂)-Alkyl, oder
2. -C(O)-N(R⁷)-R⁸, worin R⁷ und R⁸ unabhängig voneinander für Wasserstoffatom, -(C₁-C₄)-Alkyl-OH, -O-(C₁-C₄)-Alkyl oder - (C₁-C₄)-Alkyl stehen, steht
R4 für
1. einen Heteroarylrest aus der Gruppe der ungesättigten, teilweise gesättigten oder vollständig gesättigte Ringe steht, die sich von Pyridin, Pyrazin, Pyrimidin, Pyridazin, Pyrrol, Furan, Thiophen, Imidazol, Pyrazol, Oxazol, Isoxazol, Thiazol, Triazol oder Isothiazol ableiten, wobei der Heteroarylrest unsubstituiert ist oder ein-, zwei- oder dreifach unabhängig voneinander substituiert ist durch -(C₁-C₄)-Alkyl, - (C₁-C₄)-Alkoxy, F, Cl, J, Br, Nitro, Amino, Trifluormethyl, Hydroxyl, Hydroxy-(C₁-C₄)-alkyl, Methylendioxy, Ethylendioxy, Formyl, Acetyl, Cyano, Hydroxycarbonyl, Aminocarbonyl oder -(C₁-C₄)-Alkoxycarbonyl, oder
2. Phenyl steht, und Phenyl unsubstituiert ist oder ein-, zwei- oder dreifach unabhängig voneinander substituiert ist durch F, Cl, J, Br, CF₃, - OH, -(C₁-C₄)-Alkyl oder -(C₁-C₄)-Alkoxy.

3. Verbindung der Formel IV gemäß Anspruch 1, wobei die Verbindung der Formel IV 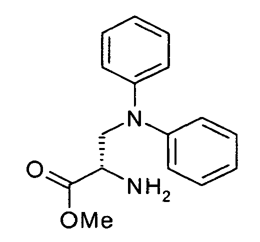 ist, worin Me für Methyl steht.

4. Verbindung der Formel

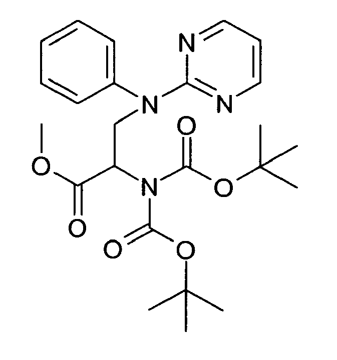

## Claims

1. A compound of the formula IV, 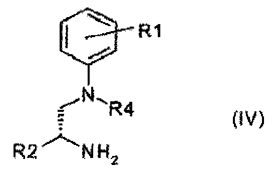 where
R1 is
1. hydrogen atom,
2. F, Cl, I or Br,
3. -(C₁-C₄)-alkyl,
4. -CN,
5. -CF₃,
6. -OR⁵, in which R⁵ is hydrogen atom or -(C₁-C₄)-alkyl,
7. -N(R⁵)-R⁶, in which R⁵ and R⁶ are, independently of each other, hydrogen atom or -(C₁-C₄)-alkyl,
8. -C(O)-R⁵, in which R⁵ is hydrogen atom or -(C₁-C₄)-alkyl, or
9. -S(O)ₓ-R⁵, in which x is the integer zero, 1 or 2, and R⁵ is hydrogen atom or-(C₁-C₄)-alkyl,
R2 is
1. a heteroaryl radical from the group 3-hydroxypyrro- 2,4-dione, imidazole, imidazolidine, imidazoline, indazole, isothiazole, isothiazolidine, isoxazole, 2-isoxazolidine, isoxazolidine, isoxazolone, morpholine, oxazole, 1,3,4-oxadiazole, oxadiazolidinedione, oxadiazolone, 1,2,3,5-oxathiadiazole-2-oxide, 5-oxo-4,5-dihydro[1,3,4]- oxadiazole, 5-oxo-1,2,4-thiadiazole, piperazine, pyrazine, pyrazole, pyrazoline, pyrazolidine, pyridazine, pyrimidine, tetrazole, thiadiazole, thiazole, thiomorpholine, triazole or triazolone, and
the heteroaryl radical is unsubstituted or is substituted, once, twice or three times, independently of each other, by
1.1 -C(O)-R₅, in which R⁵ is hydrogen atom or -(C₁-C₄)-alkyl,
1.2 -(C₁-C₄)-alkyl,
1.3 -O-R⁵, in which R⁵ is hydrogen atom or-(C₁-C₄)-alkyl,
1.4 -N(R⁵)-R⁶, in which R⁵ and R⁶ are, independently of each other, hydrogen atom or -(C₁-C₄-alkyl),
1.5 halogen, or
1.6 keto radical,
2. -C(O)-R⁵, in which R⁵ is hydrogen atom or-(C₁-C₄-alkyl),
3. -C(O)-OR⁵, in which R⁵ is hydrogen atom or -(C₁-C₄-alkyl), or
4. -C(O)-N(R⁷)-R⁸, in which R⁷ and R⁸ are, independently of each other, hydrogen atom, -(C₁-C₄)-alkyl-OH, -O-(C₁-C₄)-alkyl or -(C₁-C₄-alkyl),
R4 is
1. a heteroaryl radical from the group pyrrole, furan, thiophene, imidazole, pyrazole, oxazole, isoxazole, thiazole, isothiazole, tetrazole, 1,2,3,5-oxathiadiazo!e-2-oxides, triazolones, oxadiazolone, isoxazolone, oxadiazolidinedione, triazole, 3-hydroxypyrro-2,4-diones, 5-oxo-1,2,4-thiadiazoles, pyridine, pyrazine, pyrimidine, indole, isoindole, indazole, phthalazine, quinoline, isoquinoline, quinoxaline, quinazoline, cinnoline, β-carboline and benzofused, cyclopenta derivatives or cyclohexa derivatives of these heteroaryl radicals
where the heteroaryl radical is unsubstituted or is substituted, once, twice or three times, independently of each other, by -(C₁-C₅)-alkyl, -(C₁-C₅)-alkoxy, halogen, nitro, amino, trifluoromethyl, hydroxyl, hydroxy-(C₁-C₄)-alkyl, methylene- dioxy, ethylenedioxy, formyl, acetyl, cyano, hydroxycarbonyl, aminocarbonyl or -(C₁-C₄)-alkoxycarbonyl, or
2. an aryl radical from the group phenyl, naphthyl, 1-naphthyl, 2-naphthyl, biphenylyl, 2-biphenylyl, 3-biphenylyl and 4-biphenylyl, anthryl or fluorenyl, and
the aryl radical is unsubstituted or is substituted, once, twice or three times, independently of each other, by -(C₁-C₅)- alkyl, -(C₁-C₅)-alkoxy, halogen, nitro, amino, trifluoromethyl, hydroxyl, hydroxy-(C₁-C₄)-alkyl, methylenedioxy, ethylenedioxy, formyl, acetyl, cyano, hydroxycarbonyl, aminocarbonyl or -(C₁-C₄)-alkoxycarbonyl.

2. The compound of the formula IV as claimed in claim 1, where
R1 is
1. hydrogen atom,
2. F, Cl, I or Br,
3. -(C₁-C₄)-alkyl,
4. -CN,
5. -CF₃,
6. -OR⁵ in which R⁵ is hydrogen atom or -(C₁-C₄)-alkyl,
7. -N(R⁵)-R⁶, in which R⁵ and R⁶ are, independently of each other, hydrogen atom or -(C₁-C₄)-alkyl,
8. -C(O)-R⁵, in which R⁵ is hydrogen atom or -(C₁-C₄)- alkyl, or
9. -S(O)ₓ-R⁹, in which x is the integer zero, 1 or 2, and R⁵ is hydrogen atom or -(C₁-C₄)-alkyl,
R2 is
1. a heteroaryl radical from the group imidazole, isothiazole, isoxazole, 2-isoxazolidine, isoxazolidine, isoxazolone, 1,3,4-oxadiazole, oxadiazolidinedione, 1,2,3,5-oxadiazolone, oxazole, 5-oxo-4,5-dihydro[1,3,4]- oxadiazole, tetrazole, thiadiazole, thiazole, triazole or triazolone, and
the heteroaryl radical is unsubstituted or is substituted, once, twice or three times, independently of each other, by
1.1 keto radical,
1.2 F, Cl, I or Br or
1.3 -(C₁-C₂)-alkyl, or
2. -C(O)-N(R⁷)-R⁸, in which R⁷ and R⁸ are, independently of each other, hydrogen atom, -(C₁-C₄)- alkyl-OH, -O-(C₁-C₄)-alkyl or -(C₁-C₄)-alkyl,
R4 is
1. a heteroaryl radical from the group of the unsaturated, partially saturated or completely saturated rings which are derived from pyridine, pyrazine, pyrimidine, pyridazine, pyrrole, furan, thiophene, imidazole, pyrazole, oxazole, isoxazole, thiazole, triazole or isothiazole,
where the heteroaryl radical is unsubstituted or is substituted, once, twice or three times, independently of each other, by -(C₁-C₄)-alkyl, -(C₁-C₄)-alkoxy, F, Cl, I, Br, nitro, amino, trifluoromethyl, hydroxyl, hydroxy-(C₁-C₄)-alkyl, methylenedioxy, ethylenedioxy, formyl, acetyl, cyano, hydroxycarbonyl, aminocarbonyl or -(C₁-C₄)-alkoxycarbonyl, or
2. phenyl, and phenyl is unsubstituted or is substituted, once, twice or three times, independently of each other, by F, Cl, I, Br, CF₃, -OH, -(C₁-C₄)-alkyl or -(C₁-C₄)-alkoxy.

3. The compound of the formula IV as claimed in claim 1, where the compound of the formula IV is 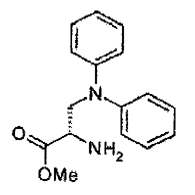 where Me is methyl.

4. A compound of the formula

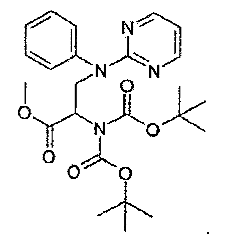

## Revendications

1. Composé de formule IV, 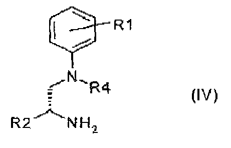 dans lequel
R¹ est
1. un atome d'hydrogène,
2. F, Cl, I ou Br,
3. un alkyle en C₁-C₄,
4. -CN,
5. -CF₃ ,
6. -OR⁵, dans lequel R⁵ est un atome d'hydrogène ou un alkyle en C₁-C₄,
7. -N(R⁵)-R⁶, dans lequel R⁵ et R⁶ sont, indépendamment l'un de l'autre, un atome d'hydrogène ou un alkyle en C₁-C₄,
8. -C(O)-R⁵, dans lequel R⁵ est un atome d'hydrogène ou un alkyle en C₁-C₄, ou
9. -S(O)ₓ-R⁵, dans lequel x est le nombre entier zéro, 1 ou 2, et R⁵ est un atome d'hydrogène ou un alkyle en C₁-C₄,
R² est
1. un radical hétéroaryle issu du groupe consistant en la 3-hydroxypyrro-2,4-dione, l'imidazole, l'imidazolidine, l'imidazoline, l'indazole, l'isothiazole, l'isothiazolidine, l'isoxazole, la 2-isoxazolidine, l'isoxazolidine, l'isoxazolone, la morpholine, l'oxazole, le 1,3,4-oxadiazole, l'oxadiazolidinedione, l'oxadiazolone, le 2-oxyde de 1,2,3,5-oxathiadiazole, le 5-oxo-4,5-dihydro[1,3,4]oxadiazole, le 5-oxo-1,2,4-thiadiazole, la pipérazine, la pyrazine, le pyrazole, la pyrazoline, la pyrazolidine, la pyridazine, la pyrimidine, le tétrazole, le thiadiazole, le thiazole, la thiomorpholine, le triazole ou la triazolone, et
le radical hétéroaryle est non substitué ou est substitué, une fois, deux fois ou trois fois, indépendamment des autres, par
1.1 -C(O)-R⁵, dans lequel R⁵ est un atome d'hydrogène ou un alkyle en C₁-C₄,
1.2 un alkyle en C₁-C₄,
1.3 -O-R⁵, dans lequel R⁵ est un atome d'hydrogène ou un alkyle en C₁-C₄,
1.4 -N(R⁵)-R⁶, dans lequel R⁵ et R⁶ sont, indépendamment l'un de l'autre, un atome d'hydrogène ou un alkyle en C₁-C₄,
1.5 un halogène, ou
1.6 un radical cétonique,
2. -C(O)-R⁵, dans lequel R⁵ est un atome d'hydrogène ou un alkyle en C₁-C₄,
3. -C(O)-OR⁵, dans lequel R⁵ est un atome d'hydrogène ou un alkyle en C₁-C₄, ou
4. -C(O)-N(R⁷)-R⁸, dans lequel R⁷ et R⁸ sont, indépendamment l'un de l'autre, un atome d'hydrogène, un -(alkyle en C₁-C₄)-OH, un -O-(alkyle en C₁-C₄) ou un alkyle en C₁-C₄,
R⁴ est
1. un radical hétéroaryle issu du groupe consistant en le pyrrole, le furane, le thiophène, l'imidazole, le pyrazole, l'oxazole, l'isoxazole, le thiazole, l'isothiazole, le tétrazole, les 2-oxydes de 1,2,3,5-oxathiadiazole, les triazolones, l'oxadiazolone, l'isoxazolone, l'oxadiazolidinedione, le triazole, les 3-hydroxypyrro-2,4-diones, les 5-oxo-1,2,4-thiadiazoles, la pyridine, la pyrazine, la pyrimidine, l'indole, l'isoindole, l'indazole, la phtalazine, la quinoline, l'isoquinoline, la quinoxaline, la quinazoline, la cinnoline, la β-carboline et les dérivés fusionnés avec un benzo, cyclopenta ou cyclohexa de ces radicaux hétéroaryle,
où le radical hétéroaryle est non substitué ou est substitué, une fois, deux fois ou trois fois, indépendamment des autres, par un alkyle en C₁-C₅, un alcoxy en C₁-C₅, un halogène, un nitro, un amino, un trifluorométhyle, un hydroxyle, un hydroxy-(alkyle en C₁-C₄), un méthylènedioxy, un éthylènedioxy, un formyle, un acétyle, un cyano, un hydroxycarbonyle, un aminocarbonyle ou un alcoxycarbonyle en C₁-C₄, ou
2. un radical aryle issu du groupe consistant en le phényle, le naphtyle le 1-naphtyle, le 2-naphtyle, le biphénylyle, le 2-biphénylyle, le 3-biphénylyle, le 4-biphénylyle, l'anthryle ou le fluorényle, et
le radical aryle est non substitué ou est substitué, une fois, deux fois ou trois fois, indépendamment des autres, par un alkyle en C₁-C₅, un alcoxy en C₁-C₅, un halogène, un nitro, un amino, un trifluorométhyle, un hydroxyle, un hydroxy-(alkyle en C₁-C₄), un méthylènedioxy, un éthylènedioxy, un formyle, un acétyle, un cyano, un hydroxycarbonyle, un aminocarbonyle ou un alcoxycarbonyle en C₁-C₄.

2. Composé de formule IV selon la revendication 1, dans lequel
R¹ est
1. un atome d'hydrogène,
2. F, Cl, I ou Br,
3. un alkyle en C₁-C₄,
4. -CN,
5. -CF₃ ,
6. -OR⁵, dans lequel R⁵ est un atome d'hydrogène ou un alkyle en C₁-C₄,
7. -N(R⁵)-R⁶, dans lequel R⁵ et R⁶ sont, indépendamment l'un de l'autre, un atome d'hydrogène ou un alkyle en C₁-C₄,
8. -C(O)-R⁵, dans lequel R⁵ est un atome d'hydrogène ou un alkyle en C₁-C₄, ou
9. -S(O)ₓ-R⁵, dans lequel x est le nombre entier zéro, 1 ou 2, et R⁵ est un atome d'hydrogène ou un alkyle en C₁-C₄,
R² est
1. un radical hétéroaryle issu du groupe consistant en l'imidazole, l'isothiazole, l'isoxazole, la 2-isoxazolidine, l'isoxazolidine, l'isoxazolone, le 1,3,4-oxadiazole, l'oxadiazolidinedione, la 1,2,3,5-oxadiazolone, l'oxazole, le 5-oxo-4,5-dihydro-[1,3,4]oxadiazole, le tétrazole, le thiadiazole, le thiazole, le triazole ou la triazolone, et
le radical hétéroaryle est non substitué ou est substitué, une fois, deux fois ou trois fois, indépendamment des autres, par
1.1 un radical cétonique,
1.2 F, Cl, I ou Br, ou
1.3 un alkyle en C₁-C₂, ou
2. -C(O)-N(R⁷)-R⁸, dans lequel R⁷ et R⁸ sont, indépendamment l'un de l'autre, un atome d'hydrogène, un -(alkyle en C₁-C₄)-OH, un -O-(alkyle en C₁-C₄) ou un alkyle en C₁-C₄,
R⁴ est
1. un radical hétéroaryle issu du groupe consistant en les cycles insaturés, partiellement saturés ou complètement saturés qui sont dérivés de la pyridine, de la pyrazine, de la pyrimidine, de la pyridazine, du pyrrole, du furane, du thiophène, de l'imidazole, du pyrazole, de l'oxazole, de l'isoxazole, du thiazole, du triazole ou de l'isothiazole,
où le radical hétéroaryle est non substitué ou est substitué, une fois, deux fois ou trois fois, indépendamment des autres, par un alkyle en C₁-C₄, un alcoxy en C₁-C₄, F, Cl, I, Br, un nitro, un amino, un trifluorométhyle, un hydroxyle, un hydroxy-(alkyle en C₁-C₄), un méthylènedioxy, un éthylènedioxy, un formyle, un acétyle, un cyano, un hydroxycarbonyle, un aminocarbonyle, ou un alcoxycarbonyle en C₁-C₄, ou
2. un phényle, et le phényle est non substitué ou est substitué une fois, deux fois ou trois fois, indépendamment des autres, par F, Cl, I, Br, CF₃, -OH, un alkyle en C₁-C₄ ou un alcoxy en C₁-C₄.

3. Composé de formule IV selon la revendication 1, le composé de formule IV étant 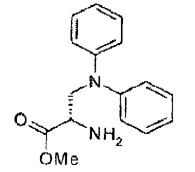 dans lequel Me représente methyl.

4. composé de formule
